# EUROPÄISCHE PATENTSCHRIFT

(11) **EP 2 627 617 B1**
(45) Veröffentlichungstag und Bekanntmachung des Hinweises auf die Patenterteilung: **07.01.2015**
(21) Anmeldenummer: 11767004.2
(22) Anmeldetag: 07.10.2011
(51) Int. Cl.: C07B 63/04, C07D 251/24, C08K 5/3492, C09K 15/30

(54) **SPEZIELLE UV-ABSORBER FÜR HÄRTBARE UV-SCHUTZ BESCHICHTUNGEN**
SPECIAL UV ABSORBERS FOR CURABLE UV PROTECTIVE COATINGS
ABSORBEURS D'UV SPÉCIAUX POUR REVÊTEMENTS ANTI-UV DURCISSABLES

(30) Priorität: 12.10.2010 EP 10187268
(43) Veröffentlichungstag der Anmeldung: 21.08.2013
(73) Patentinhaber: Bayer Intellectual Property GmbH, 40789 Monheim am Rhein (DE)
(72) Erfinder: KOSTROMINE, Serguei, 53913 Swisttal (DE); KUHLMANN, Timo, 42799 Leichlingen (DE); OSER, Rafael, 47800 Krefeld (DE)
(74) Vertreter: BIP Patents
(86) Internationale Anmeldenummer: PCT/EP2011/067572
(87) Internationale Veröffentlichungsnummer: WO 2012/049091

(56) Entgegenhaltungen:
- DE-A1- 19 739 781

## Beschreibung

Die vorliegende Erfindung betrifft organische UV-Absorber, die zwei oder mehrere polymerisierbare Acrylat oder Methacrylat-Gruppen im Molekül aufweisen, Beschichtungsmittel enthaltend solche UV-Absorber sowie daraus hergestellte Beschichtungen und sowie mit diesen beschichtete Substrate.

Für Außenanwendungen müssen transparente Kunststoffartikel, wie z.B. Platten, Folien oder Extrusionsformkörper, vor allem mittels UV-Schutz vor der aggressiven Sonnenstrahlung und mittels Kratzfestausrüstung vor mechanischen Einwirkungen geschützt werden. Ein gängiges Verfahren dafür ist die obere und manchmal auch die einzige Schutzschicht, die kratzfest sein muss, zusätzlich mit UV-Schutzfunktion zu versehen und dafür mit einer wesentlichen Menge von UV-Absorbern zu bestücken (vgl. DE-A 10 2006 016 642). Die klassischen UV-Absorber wirken allerdings in den Schutzschichten als Weichmacher und verringern die mechanische Beständigkeit der Schicht.

Typische UV-Absorber-Klassen sind beispielsweise Biphenyl-substituierte Triazine (vgl. WO-A 2006/108520). Diese Substanzklasse zeigt eine hervorragende Absorptionswirkung bei 320 - 380 nm und gleichzeitig eine sehr hohe eigene UV-Stabilität (WO 2000/066675 A1, US-A 6,225,384).

Darüber hinaus existieren nicht unbedeutende Kompatibilitätsprobleme zwischen dem UV-Absorber und den Komponenten des Beschichtungsmittels. Für kratzfeste Sol-Gel-Beschichtungen, die außerordentlich gute Kratzfestigkeit aufweisen, ist es beispielsweise sehr schwierig, einen kompatiblen UV-Absorber zu finden, da die in der Regel hocharomatischen UV-Absorber in den polaren Sol-Gel-Lackmischungen kaum löslich sind. Zwar konnten die Löslichkeit von UV-Absorbern, wie z.B. der Klasse der 2-Hydroxy-benzophenone (US 5,679,820; US 5,391,795), in Sol-Gel-Lacksystemen durch gezieltes Modifizieren der UV-Absorber-Moleküle mittels Einbau von Trialkoxysilan-Gruppen erhöht werden, jedoch kann die Einführung solcher großen organischen Moleküle in eine anorganische Silica-Matrix in signifikanter Konzentration von mehreren Gewichtsprozenten aber zu Abstrichen von Härte und Kratzfestigkeit der Beschichtung führen, so dass sich das optimale Potenzial von Sol-Gel-Lackierungen hinsichtlich ihre mechanischen Beständigkeit nicht mehr ausschöpfen lässt.

Eine mögliche Lösung dieses Problems könnte die Trennung von Schichten mit UV- und mechanischer Schutzfunktionen sein, wobei die erste "weiche" rein-organische Schicht ausreichend UV-Absorber beinhaltet und die zweite äußere möglichst harte Schicht Kratzer und Abrieb abwehrt. Solche Ansätze sind bereits bekannt. Beispielsweise ist die Schicht von Haftvermittlern (Primern) bei Sol-Gel-Lackierungen oft mit UV-Absorbern versehen (vgl. z.B. US 5,041,313; US 5,869,185; US 5,981,073; US 6,350,521; DE-A 10 2007 050192). Solche Primer-Schichten enthalten im Wesentlichen lösliche Polymethacrylate, sind in der Regel dünn, dürfen aber gleichzeitig auch nicht zu weich sein. Letzteres führt wiederum dazu, dass man die notwendige Menge des klassischen UV-Absorbers, die zum Erreichen eines ausreichenden UV-Schutzes erforderlich wäre, solchen Schichten nicht zumischen kann. Das Ergebnis des mangelnden UV-Schutzes der einzelnen Schicht versucht man dann, durch Zugabe von UV-Absorbern in allen vorhandenen Schutz- und Zwischenschichten (Primer- und Klarlackschichten, vgl. z.B. US 4,410,594) auszugleichen und gegebenenfalls darüber hinaus auch noch dem zu schützenden Substrat zuzumischen (z.B. US-A 2009-0258978), was wiederum zu der Problematik der Funktion der UV-Absorbers als Weichmacher führt. Da die klassischen UV-Absorber zudem kristalline organische Substanzen sind, die alleine keine filmbildenden Eigenschaften besitzen und deswegen in einem filmbildenden Bindemittel gelöst werden müssen, werden separate UV-schützende Primer-Schichten in der Regel zu dick und oftmals zu weich, um überhaupt wesentlich zum UV-Schutz beitragen zu können.

Es bestand demnach weiterhin Bedarf nach geeigneten UV-Absorbern, mit denen Primerschichten mit ausreichender UV-Schutzwirkung hergestellt werden können, die die vorangehend genannten Nachteile nicht aufweisen.

Demnach bestand die Aufgabe, die der vorliegenden Erfindung zugrunde lag, darin, UV-Absorber aufzufinden, mit denen dünne und nicht zu weiche Primerschichten mit ausreichender UV-Schutzwirkung hergestellt werden können, so dass auf eine weitere Zugabe von UV-Absorbern in der kratzfesten Deckschicht verzichtet werden kann oder eine weitere Zugabe von UV-Absorbern in der kratzfesten Deckschicht nicht mehr in solchen Mengen erforderlich ist, dass deren Kratzfestfunktion beeinträchtigt wird.

Diese Aufgabe wurde mit der vorliegenden Erfindung durch die Bereitstellung von UV-Absorbern der Substandklasse der s-Triazine, bevorzugt Biphenyl-substituierten s-Triazine gelöst, die zwei oder mehrere polymerisierbare Acrylat- und/oder Methacrylat-Gruppen aufweisen, welche an einem offenkettigen Substituenten des harten aromatischen Farbstoff-Kerns hängen.

Gegenstand der vorliegenden Erfindung sind Verbindungen der allgemeinen Formel (I)

A - X (- T)ₙ (I),

wobei
- A: für 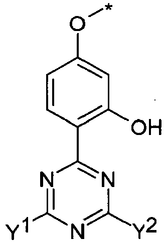 steht, worin
Y¹ und Y² unabhängig voneinander für Substituenten der generellen Formel 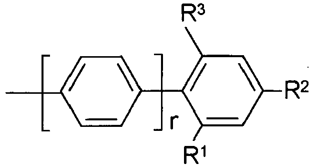 stehen, worin
r für 0 oder 1, bevorzugt für 1 steht,
R¹, R², R³ unabhängig voneinander für H, OH, C₁₋₂₀-Alkyl, C₄₋₁₂-Cycloalkyl, C₂₋₂₀-Alkenyl, C₁₋₂₀-Alkoxy, C₄₋₁₂-Cycloalkoxy, C₂₋₂₀-Alkenyloxy, C₇₋₂₀-Aralkyl, Halogen, -C≡N, C₁₋₅-Haloalkyl, -SO₂R', -SO₃H, -SO₃M (M = Alkalimetall), -COOR', -CONHR', -CONR'R", -OCOOR', -OCOR', - OCONHR', (Meth)acrylamino, (Meth)acryloxy, gegebenenfalls durch C₁₋₁₂-Alkyl, C₁₋₁₂-Alkoxy, CN und/oder Halogen substituiertes C₆₋₁₂-Aryl oder gegebenenfalls durch C₁₋₁₂-Alkyl, C₁₋₁₂-Alkoxy, CN und/oder Halogen substituiertes C₃₋₁₂-Heteroaryl stehen, worin
M für ein Alkalimetallkation steht,
R' und R" für H, C₁₋₂₀-Mkyl, C₄₋₁₂-Cycloalkyl, gegebenenfalls durch C₁₋₁₂-Alkyl, C₁₋₁₂-Alkoxy, CN und/oder Halogen substituiertes C₆₋₁₂-Aryl oder gegebenenfalls durch C₁₋₁₂-Alkyl, C₁₋₁₂-Alkoxy, CN und/oder Halogen substituiertes C₃₋₁₂-Heteroaryl stehen,
- X: für einen gegebenenfalls substituierten linearen oder verzweigten Linker aus Kohlenstoff, Sauerstoff, Stickstoff, Schwefel, Phosphor und/oder Silizium in der Kette steht,
- T: für einen Acrylat-Rest -O-(C=O)-CH=CH₂ oder einen Methacrylat-Rest -O-(C=O)-C(CH₃)=CH₂ steht, und
- n: für eine ganze Zahl von 2 bis 5 steht.

Erfindungsgemäß sind gemäß der allgemeinen Formel (I) mindestens zwei Reste T an den Linker X gebunden.

Die erfindungsgemäßen UV-Absorber weisen selbst filmbildende Eigenschaften auf. Bei Auftragung auf die Oberfläche des Kunststoffs bilden sie eine amorphe, transparente und klare Beschichtung und neigen nicht zur Kristallisation. Die mit den erfindungsgemäßen UV-Absorbern hergestellten Beschichtungen sind zudem aufgrund der polymerisierbaren Acrylat- und/oder Methacrylat-Gruppen thermisch oder UV-härtbar oder durch eine andere gängige Härtungsmethode (z.B. Elektonenstrahl-, Plasma-Härten etc.) härtbar. Daher ermöglichen die erfindungsgemäßen UV-Absorber die Herstellung dünner und fester Schichten, die ausreichend UV-Strahlung absorbieren und für kratzfeste Lacke, bevorzugt Sol-Gel-Lacke, eine beständige Grundlage (Primerschicht) bieten können. Aufgrund der ausreichenden UV-Schutzwirkung dieser Primer-Schicht ist eine Zugabe weiterer UV-Absorber zu einer solchen äußeren Kratzfestlackierung nicht mehr oder zumindest nicht mehr in größeren Mengen erforderlich, so dass auch keine Beeinträchtigung ihrer mechanischen Beständigkeit in Kauf genommen werden muss.

Triazine mit (Meth)acrylat-Gruppen wurden bereits mehrmals beschrieben (DE-A 197 39 781; EP-A 0 706 083; US 6,500,887; unveröffentlichte deutsche Patentanmeldung DE 10 2009 019493.2). In EP-A 0 706 083 wurden mehrere Triazine mit jeweils nur einer Methacrylat-Gruppe beschrieben. Diese Verbindungen sind jedoch kristalline Sunstanzen, die als härtende Lacke nicht eingesetzt werden können. Solche Substanzen wurden zur Herstellung UV-absorbierender Copolymere verwendet. Kristallines Triazin mit einer Methacrylat-Gruppe wird auch in US 6,500,887 beschrieben, das auf klassische Weise dem zu schützenden Kunststoff zugegeben wurde. In DE-A 197 39 781, EP-A 0 706 083, US 6,500,887 und der noch nicht veröffentlichten deutschen Patentanmeldung DE 10 2009 019493.2 wurden zudem auch Triazine beschrieben, die mehrere Methacrylat-Gruppen beinhalten. Solche Gruppen sind aber in den Molekülen derart verteilt, dass an jedem einzelnen Substituenten des harten aromatischen Farbstoff-Kerns ausschließlich eine Methacrylat-Gruppe hängen darf. Die offenbarten Anwendungswege solcher Substanzen gehen nicht über die für die nur eine Methacrylat-Gruppe enthaltenden Verbindungen hinaus. Der Einsatz solcher Substanzen selbst als härtende, filmbildende Verbindungen für UV-Schutzlacke zum Schutz von Kunststoffen ist nicht beschrieben.

Die erfindungsgemäßen Verbindungen sind daher neu.

Die erfindungsgemäßen Verbindungen weisen bevorzugt ein UV-Absorptionsmaximum zwischen 300-340 nm auf.

Bevorzugt steht X in den Verbindungen der allgemeinen Formel (I) für einen gegebenenfalls substituierten linearen oder verzweigten Linker, wobei zwischen dem O-Atom der Gruppe A und jeder T-Gruppe eine Kette aus mindestens 4 Atomen ausgewählt aus Kohlenstoff, Sauerstoff, Stickstoff, Schwefel, Phosphor und/oder Silizium in der Kette besteht.

Bevorzugt handelt es sich bei den Verbindungen der allgemeinen Formel (I) um solche der allgemeinen Formel (I-1) 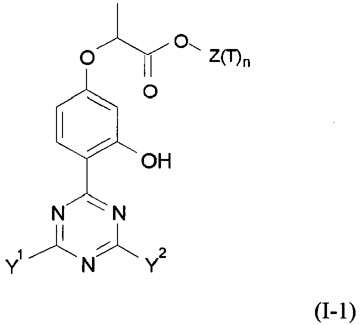 worin
- Z: für einen gegebenenfalls substituierten linearen oder verzweigten C₁₋₂₀-Alkylen-Rest oder C₁₋₂₀-Alkylenether-Rest steht, und

T, n, Y¹ und Y² die vorangehend für die Verbindungen der allgemeinen Formel (I) genannte Bedeutung haben.

Von den Verbindungen der allgemeinen Formel (I-1) sind Verbindungen der allgemeinen Formel (II) besonders bevorzugt

A-C(R⁴)H-C(=O)-O-CH₂-C(R⁵)p(CH₂-T)q (II),

worin
- R⁴: für H oder C₁₋₂₀-Alkyl steht,
- R⁵: für H, C₁₋₂₀-Alkyl oder -CH₂-OH steht
- p: für 0 oder 1 steht,
- q: für 3 - p steht.

Weiterhin sind von den Verbindungen der allgemeinen Formel (I) bzw. (I-1) die Verbindungen der allgemeinen Formel (III) bevorzugt

A-C(R⁴)H-C(=O)-O-CH₂-CH(T)-CH₂-T (III)

worin
- R⁴: für H oder C₁₋₂₀-Alkyl steht.

Weiterhin sind von den Verbindungen der allgemeinen Formel (I) bzw. (I-1) die Verbindungen der allgemeinen Formel (IV) bevorzugt

A-C(R⁴)H-C(=O)-O-CH₂-C(R⁵)m(CH₂-T)n-CH₂-O-CH₂-C(R⁵)p(CH₂-T)q (IV)

worin
- R⁴: für H oder C₁₋₂₀-Alkyl steht,
- R⁵: für -CH₂-OH steht
- m: für 0 oder 1 steht,
- n: für 2 - m steht und
- p: für 0, 1 oder 2 steht,
- q: für 3 - p steht.

In den Formeln (II), (III) und (IV) haben A und T die vorangehend für die Verbindungen der allgemeinen Formel (I) genannte Bedeutung.

In bevorzugten Ausführungsformen steht in den Substituenten Y¹ und Y² in den Formeln (I), (I-1) oder (II) bis (IV) r jeweils für 1.

In bevorzugten Ausführungsformen stehen in den Substituenten Y¹ und Y² in den Formeln (I), (I-1) oder (II) bis (IV) die Reste R¹, R² und R³ jeweils für H.

Besonders bevorzugt stehen die Substituenten Y¹ und Y² in den Formeln (I), (I-1) oder (II) bis (IV) gleichzeitig für

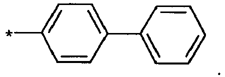

Ganz besonders bevorzugt sind von den Verbindungen mit der allgemeinen Formel (I) bzw. (I-1) folgende Verbindungen der Formeln (I-1-1) bis (I-1-12) handelt

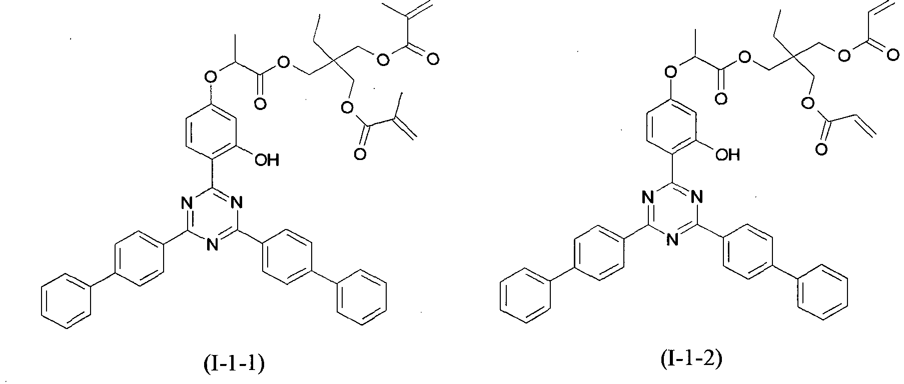

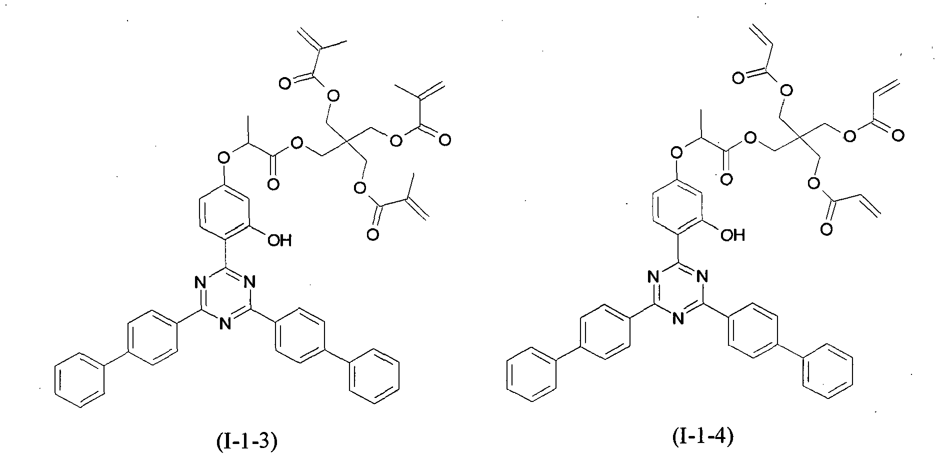

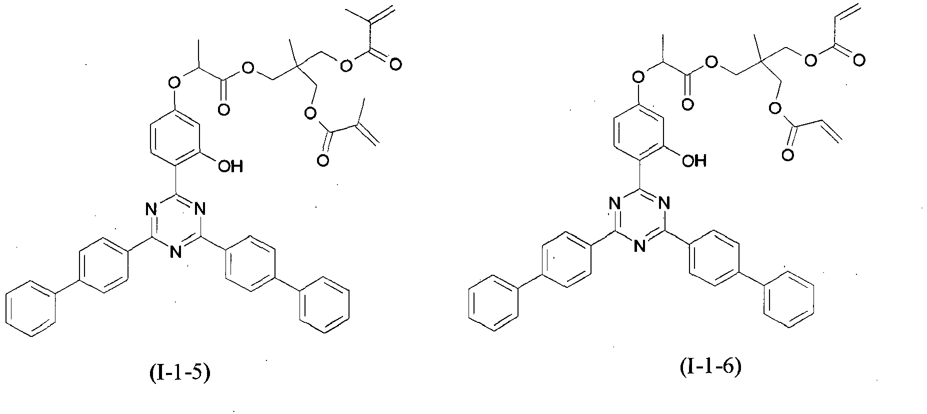

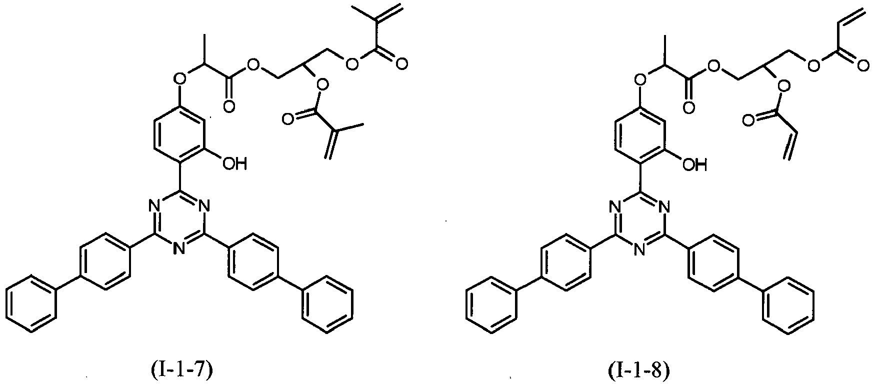

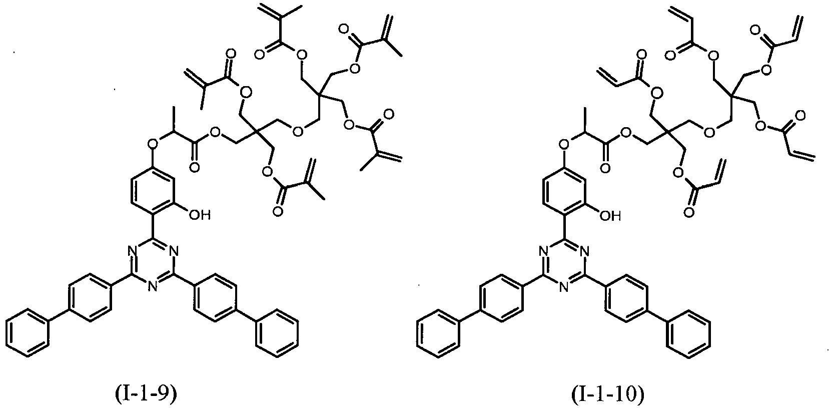

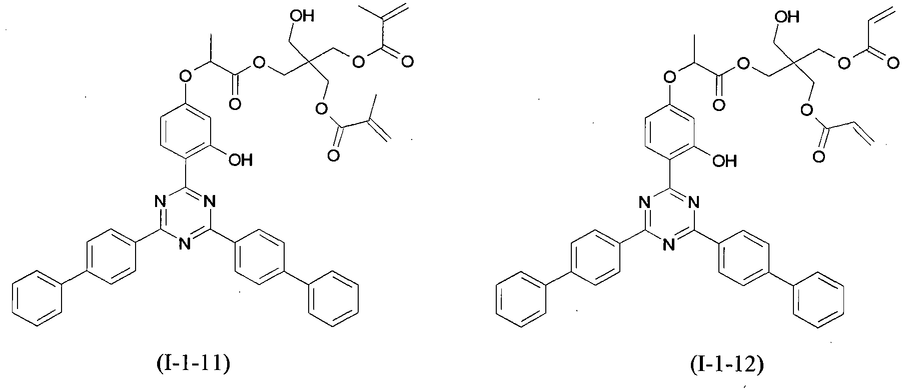

Ein weiterer Gegenstand der Erfindung sind Beschichtungszusammensetzungen, die wenigstens eine der erfindungsgemäßen UV-absorbierenden Verbindungen der allgemeinen Formel (I) enthalten.

Eine bevorzugte Formulierung der erfindungsgemäßen Beschichtungszusammensetzung für das Auftragen der Verbindungen der allgemeinen Formel (I) auf die Oberfläche der Kunststoffe ist eine Lösung in gängigen organischen Lösungsmitteln.

Vorzugsweise enthalten die erfindungsgemäßen Beschichtungszusammensetzungen daher wenigstens ein organisches Lösemittel. Geeignete Lösemittel sind beispielsweise aromatische Lösemittel, Ketone, Alkohole, Ether oder Ester, wie z.B. Alkylacetate. Besonders bevorzugt sind Toluol, Butylacetat, Diacetonalkohol und 2-Methoxypropanol. Das Lösemittel soll bevorzugt an den zu beschichtenden Kunststoff angepasst werden, damit oberflächliche Zerstörung beispielsweise durch Anlösen vermieden wird. Für das Beschichten von Substraten enthaltend Polycarbonate oder Copolycarbonate ist Diacetonalkohol oder 2-Methoxypropanol als Lösemittel besonders bevorzugt.

Die erfindungsgemäßen Beschichtungszusammensetzungen können bevorzugt zusätzlich die Initiatoren der Polymerisation enthalten. Bevorzugte Initiatoren sind thermisch oder UV-getriebene Initiatoren. Übliche oberhalb von 50°C in Radikale zerfallende Peroxide, wie Diacylperoxyde, Peroxydicarbonate, Peroxyester, Perketale, Hydroxyperoxyde, Ketoperoxide und Dialkylperoxyde können als thermisch Initiatoren eingesetzt werden. Geeignete thermische Initiatoren sind auch typische Azo-Initiatoren.

Geeignete UV-getriebene Initiatoren (Photoinitiatoren) besitzen vorzugsweise eine hohe photochemische Reaktivität und eine Absorptionsbande im nahen UV-Bereich (>300 nm und besonders bevorzugt >350 nm).

Geeignete Photoinitiatoren sind vorzugsweise solche ausgewählt aus der Gruppe aus Acylphosphinoxid-Derivaten und α-Aminoalkylphenon-Derivaten.

Bevorzugt werden als Photoinitiatoren Bis(2,4,6-trimethylbenzo-yl)phenylphosphinoxid (Irgacure® 819 von Ciba Speciality Chemicals), (2,4,6-Trimethylbenzoyl)diphenylphosphinoxid (Lucirin® TPO Solid von der BASF AG), Bis(2,6-dimethylbenzoyl)(2,4,4-trimethylpentyl)phosphinoxid, Bis(2,6-dimethoxybenzoyl)(2,4,4-trimethylpentyl)phosphinoxid, Benzoylphosphonsäurebis(2,6-dimethylphenyl)ester (Lucirin® 8728 von der BASF AG), 2,4,6-Trimethylbenzoylethoxyphenylphosphinoxid (Lucirin® TPO-L von der BASF AG), 2-Benzyl-2-(dimethylamino)-1-(4-morpholinophenyl)-1-butanon (Irgacure® 369 von Ciba Speciality Chemicals) und 2-Methyl-1-[4-(methylthio)phenyl]-2-morpholino-1-propanon; Irgacure® 907 von Ciba Speciality Chemicals) eingesetzt.

Besonders bevorzugt werden als Photoinitiatoren Bis(2,4,6-trimethylbenzoyl)phenylphosphinoxid (Irgacure® 819 von Ciba Speciality Chemicals), 2,4,6-Trimethylbenzoylethoxyphenylphosphinoxid (Lucirin® TPO-L von der BASF AG) und 2-Methyl-1-[4-(methylthio)phenyl]-2-morpholino-1-propanon (Irgacure® 907 von Ciba Speciality Chemicals) eingesetzt.

Ebenfalls geeignet sind Mischungen dieser Photoinitiatoren untereinander sowie Mischungen der Photoinitiatoren mit anderen allgemein bekannten Photoinitiatoren wie beispielsweise α-Hydroxyalkylphenonen oder Phenylacetophenonen. Bevorzugt werden Mischungen aus Bis(2,6-dimethoxybenzoyl)(2,4,4-trimethylpentyl)phosphinoxid und (1-Hydroxycyclohexyl)phenylmethanon, vorzugsweise im Verhältnis 25 : 75 (Irgacure® 1800 von Ciba Speciality Chemicals), Mischungen aus 2,4,6-Trimethylbenzoyl-diphenyl-phosphinoxid und 2-Hydroxy-2-methyl-1-phenyl-1-propanon vorzugsweise im Verhältnis 50 : 50 (Darocur 4265 von Ciba Speciality Chemicals) oder eine Mischung aus Bis(2,6-dimethoxybenzoyl)(2,4,4-trimethylpentyl)phosphinoxid und 2-Hydroxy-2-methyl-1-phenyl-1-propanon, vorzugsweise im Verhältnis 25 : 75 (Irgacure® 1700 von Ciba Speciality Chemicals), eingesetzt.

Die erfindungsgemäßen Beschichtungszusammensetzungen können auch Stabilisatoren enthalten. Bevorzugte Stabilisatoren sind sogenannte HALS (Hindered Amine Light Stabiliser) als basische Stabilisatoren. HALS, d.h. sterisch gehinderte Amine, sind im Allgemeinen flüssige oder feste Piperidin-Derivate der allgemeinen Formel (V) 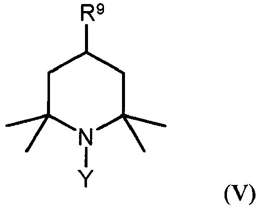 worin
- Y: für H oder CH₃ steht, und
- R⁹: für Z-R¹⁰-Z-R¹¹, 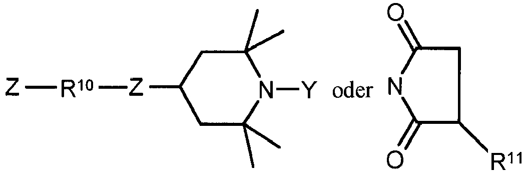 steht,
wobei
- Z: für eine divalente funktionelle Gruppe wie beispielsweise und bevorzugt -C(O)O-, -NH- oder -NHC(O)- steht,
- R¹⁰: für einen divalenten organischen Rest wie beispielsweise und bevorzugt -(CH₂)₁-, worin 1 für eine ganze Zahl von 1 bis 12, bevorzugt von 3 bis 10 steht, -C=CH-Ph-O(CH₃)-, 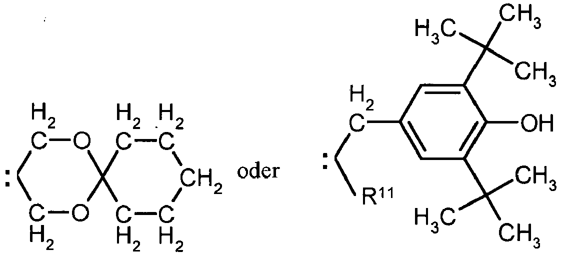 steht und
- R¹¹: für H oder C₁-C₂₀-Alkyl steht.

Sterisch gehinderte Amine wirken als Radikalfänger, die die beim Polymerabbau gebildeten Radikale abfangen. Eine generelle Übersicht über HALS-Typen bietet T. Bolle in Lackadditive, J. Bielemann, Hrsg.; Wiley-VCH: Weinheim (1998) und A. Valet in Lichtschutzmittel für Lacke, Vincentz Verlag: Hannover (1996). Bevorzugte HALS sind in der Veröffentlichung EP-A 1 308 084 sowie der DE-A 60 307 122 offenbart auf deren Kombination mit den hier vorliegenden Verbindungen der allgemeinen Formel (I) besonders hingewiesen wird.

Die erfindungsgemäßen Verbindungen der allgemeinen Formel (I) sind nicht kristallisierbar und bilden bereits ohne Zusatz von weiteren polymerisierbaren Comonomeren, Vernetzer, oligomeren oder polymeren Bindemittel und/oder Verdicker eine amorphe und transparente Beschichtung, welche durch thermisches oder UV-Härten oder durch eine weitere gängige Härtungsmethode (z.B Elektonenstrahl-, Plasma-Härten usw.) in eine feste, dünne UV-schützende Schicht überführt werden kann.

In bevorzugten Ausführungsformen der vorliegenden Erfindung enthalten die erfindungsgemäßen Beschichtungszusammensetzungen demnach keine weiteren polymerisierbaren Comonomeren, Vernetzer, oligomeren oder polymeren Bindemittel und Verdicker.

Für bestimmte Anwendungen kann es aber von Vorteil sein, dass die erfindungsgemäßen Beschichtungszusammensetzungen zusätzlich zu wenigstens einer Verbindung der allgemeinen Formel (I) auch ein oder mehrere Reaktivverdünner und/oder reaktive, d.h. polymerisierbare, oligomere oder polymere Bindemittel enthalten. Bevorzugt werden solche Reaktivverdünner und/oder oligomere oder polymere Bindemittel in einer Menge von 80 Gew.-% oder weniger bezogen auf das Gesamtgewicht der Verbindungen der allgemeinen Formel (I) zugegeben. Geeignete Reaktivverdünner sind bekannt und sind in Chemistry & Technology of UV & EB Formulation for Coatings, Inks & Paints, Vol. 2, 1991, SITA Technology, London (P.K.T: Oldring (Ed.) auf S.237-306 (Reactive Diluents) beschrieben. Bevorzugte Reaktivverdünner sind hier beispielsweise Methandioldiacrylat, 1,2-Ethandioldiacrylat, 1,3-Propandioldiacrylat, 1,2-Propandioldiacrylat, Glycerintriacrylat, 1,4-Butandioldiacrylat, 1,3-Butandioldiacrylat, 1,2,4-Butantrioltriacrylat, 1,5-Penandioldiacrylat, Neopentylglykoldiacrylat, Pentaerythritoltriacrylat, Pentaerythritoltetraacrylat, 1,6-Hexandioldiacrylat, Trimethylolpropandiacrylat, Trimethylolpropantriacrylat, Tricyclodecandimethanoldiacrylat, Diethylenglykoldiacrylat, Triethylenglykoldiacrylat, Tetraethylenglykoldiacrylat, Dipropylenglykoldiacrylat, Tripropylenglykoldiacrylat, Trimethylolpropantriethoxytriacrylat, Dipentaerythritolpentaacrylat, Dipentaerythritolhexaacrylat, Ditrimethylolpropantetraacrylat und die entsprechenden Methacrylatderivate. Besonders bevorzugt werden 1,6-Hexandioldiacrylat, Tricyclodecandimethanoldiacrylat, Trimethylolpropantriacrylat, Pentaerythritoltetraacrylat und deren Methacrylatderivate eingesetzt.

Ebenfalls geeignet als optionale reaktive polymerisierbare Komponente sind oligomere aliphatische Urethan- bzw Polyesteracrylate (reaktive oligomere oder polymere Bindemittel). Die Herstellung der geeigneten Oligomere, die zur Klasse der aliphatischen Urethanacrylate beziehungsweise der Polyesteracrylate gehören, und deren Verwendung als Lackbinder sind bekannt und sind in Chemistry & Technology of UV & EB Formulation for Coatings, Inks & Paints, Vol. 2, 1991, SITA Technology, London (P.K.T: Oldring (Ed.) auf S.73-123 (Urethane Acrylates) bzw. S.123-135 (Polyester Acrylates) beschrieben. Kommerziell erhältlich und geeignet im erfindungsgemäßen Sinne sind hier beispielsweise aliphatische Urethanacrylate wie Ebecryl® 4858, Ebecryl® 284, Ebecryl® 265, Ebecryl® 264 (Hersteller jeweils Cytec Surface Specialities), Craynor® 925 von Cray Valley, Viaktin® 6160 von Vianova Resin, Roskydal® 2258 von Bayer MaterialScience AG, Photomer 6891 von Cognis oder auch in Reaktivverdünnern gelöste aliphatische Urethanacrylate wie Laromer® 8987 (70%ig in Hexandioldiacrylat) von BASF AG, Desmolux® U680H (80%ig in Hexandioldiacrylat) von Bayer MaterialScience AG, Craynor® 945B85 (85% in Hexandioldiacrylat) und Craynor® 963B80 (80% in Hexandioldiacrylat) jeweils von Cray Valley oder auch Polyesteracrylate wie Ebecryl® 810 oder 830 von Cytec Surface Specialities.

An der Stelle der optionalen reaktiven polymerisierbaren Komponente können auch die im Handel erhältlichen fertigen UV-Lacke eingesetzt werden. Solche Lacke sind beispielsweise von der Fa. Momentive Performance Materials unter den Produktbezeichnungen UVHC3000, UVHC3000K; UVHC3000H, UVHC7000, UVHC8558 und UVHC8600 erhältlich.

Die erfindungsgemäßen Beschichtungszusammensetzungen können optional ein oder mehrere weitere Additive ausgewählt aus der Gruppe enthaltend Stabilisatoren, Verlaufsmittel, Oberflächenadditive, Pigmente, Farbstoffe, Haftvermittler, IR-Absorber und UV-Absorber verschieden von den Verbindungen der allgemeinen Formel (I) enthalten.

Die erfindungsgemäßen Beschichtungszusammensetzungen können optional auch anorganische Nanopartikel zur Erhöhung der mechanischen Beständigkeit sowie zum zusätzlichen Schutz vor UV-Strahlung enthalten.

Als Nanopartikel kommen anorganische Oxide, Mischoxide, Hydroxide, Sulfate, Carbonate, Carbide, Boride und Nitride von Elementen der II bis IV Hauptgruppe und/oder Elementen der I bis VIII Nebengruppe des Periodensystems einschließlich der Lanthanide in Frage. Bevorzugte Nanopartikel sind Siliziumoxid-, Aluminiumoxid-, Ceroxid-, Zirkonoxid-, Nioboxid-, Zinkoxid- oder Titanoxid-Nanopartikel, besonders bevorzugt sind Siliziumoxid-Nanopartikel.

Die eingesetzten Partikel weisen vorzugsweise mittlere Partikelgrößen (gemessen mittels dynamischer Lichtstreuung in Dispersion bestimmt als Z-Mittelwert) kleiner 200 nm, bevorzugt von 5 bis 100 nm, besonders bevorzugt 5 bis 50 nm auf. Bevorzugt weisen wenigstens 75%, besonders bevorzugt wenigstens 90 %, ganz besonders bevorzugt wenigstens 95% aller eingesetzten Nanopartikel die vorstehend definierten Größen auf.

Die Nanopartikel können prinzipiell sowohl in Pulverform als auch in Form von kolloiden Suspensionen oder Dispersionen in geeigneten Lösungsmitteln eingesetzt werden. Die anorganischen Nanopartikel werden bevorzugt in kolloid-disperser Form in organischen Lösungsmitteln (Organosole) verwendet. Für die Organosole geeignete Lösemittel sind beispielsweise Alkohole, wie z.B. Methanol, Ethanol, i-Propanol, Ketone, wie z.B. Aceton, 2-Butanon, Methylisobutylketon, Diacetonalkohol, Ester, wie z.B. Butylacetat, Ethylacetat, 1-Methoxy-2-propylacetat, aromatische Lösungsmittel, wie z.B. Toluol, Xylol, sowie Ether, wie z.B. 1,4-Dioxan, Ethylenglykol-n-propylether, oder beliebige Gemische solcher Lösungsmittel. Geeignete Organosole weisen einen Feststoffgehalt von 10 bis 60 Gew.-%, bevorzugt 15 bis 50 Gew.-% auf. Geeignete Organosole sind beispielsweise Siliziumdioxid Organosole wie sie z.B. unter den Handelsnamen Organosilicasol® und Suncolloid® (Nissan Chem. Am. Corp.) oder unter der Bezeichnung Highlink®NanO G (Clariant GmbH) erhältlich sind.

Die Nanopartikel können oberflächenmodifiziert sein. Vorzugsweise eignen sich solche anorganischen Partikel, die an der Oberfläche durch Silanisierung modifiziert wurden. Diese Methode ist im Prinzip literaturbekannt und beispielsweise in DE-A 19846660 oder WO-A 2003/44099 beschrieben. Weiterhin kann die Oberfläche der anorganischen Nanopartikel adsorptiv/assoziativ durch Tenside oder Blockcopolymere, wie beispielsweise in WO-A 2006/008120 bzw. Foerster, S. & Antonietti, M., Advanced Materials, 10, no. 3, (1998) 195 modifiziert werden. Bevorzugte Oberflächenmodifizierung ist die Silanisierung mit Alkoxysilanen und/oder Chlorsilanen. Besonders bevorzugt ist die partielle Modifizierung mit γ-(Meth)-acryloxypropyltri(m)ethoxysilan oder γ-Glycidoxypropyltrimethoxysilan entsprechend der WO-A 2004/035474.

Die erfindungsgemäßen Beschichtungszusammensetzungen können auf einfache Weise hergestellt werden, indem die einzelnen Komponenten Verbindung(en) der allgemeinen Formel (I), gegebenenfalls Initiator(en), Stabilisator(en), Reaktivverdünner, Bindemittel und sonstige Additive in das oder die Lösemittel gegeben und durch Rühren miteinander vermischt werden. Bevorzugt werden zuerst die Verbindung(en) der allgemeinen Formel (I) in dem oder den Lösemitteln gelöst und anschließend die weiteren Komponenten hinzugegeben. Gegebenenfalls erfolgt anschließend noch eine Reinigung mittels Filtration. Die Nanopartikel können den erfindungsgemäßen Beschichtungszusammensetzungen während oder im Anschluss an die Herstellung der zuvor beschriebenen Mischung von Komponenten zugegeben werden. Dies kann durch einfaches Einrühren der Partikel in die erfindungsgemäßen Beschichtungszusammensetzungen erfolgen. Denkbar ist jedoch auch der Einsatz erhöhter Dispergierenergie, wie beispielsweise durch Ultraschall, Strahldispergierung oder Hochgeschwindigkeitsrüher nach dem Rotor-Stator-Prinzip. Bevorzugt ist einfaches mechanisches Einrühren.

Die erfindungsgemäßen Beschichtungszusammensetzungen eignen sich zur Herstellung von Beschichtungen mit UV-Schutzwirkung. Dazu können die erfindungsgemäßen Beschichtungszusammensetzungen nach gängigen Verfahren auf entsprechende Substrate appliziert und danach unter geeigneten Bedingungen aushärtet werden.

Gegenstand der vorliegenden Erfindung ist daher auch ein Verfahren zur Beschichtung von Substraten, dadurch gekennzeichnet, dass
A) auf das Substrat eine Schicht aus einer Beschichtungszusammensetzung gemäß wenigstens einem der Ansprüche 6 bis 10 aufgebracht wird,
B) anschließend wenigstens ein Teil des Lösemittels aus der gemäß Schritt A) erhaltenen Schicht entfernt wird, und
C) anschließend die gemäß Schritt B) erhaltene Schicht ausgehärtet wird.

Die Applikation (Aufbringung) kann beispielsweise durch Tauchen, Fluten, Sprühen, Rakeln, Gießen, Spin-Coating oder Streichen erfolgen. Anschließend wird gegebenenfalls vorhandenes Lösungsmittel ganz oder teilweise entfernt, bevorzugt verdampft, und die so erhaltene Beschichtung bei Raumtemperatur oder erhöhter Temperatur, durch UV-Licht oder durch eine andere gängige Härtungsmethode (z.B. Elektonenstrahl-, Plasma-Härten etc.), bevorzugt durch UV-Licht ausgehärtet. Angaben zur Applikation nach gängigen Methoden finden sich beispielsweise in Organic Coatings: Science and Technology, John Wiley & Sons 1994, Kapitel 22, Seiten 65-82.

Die aus den erfindungsgemäßen UV-Schutzformulierungen hergestellten Beschichtungen (C) bieten einen sehr guten Schutz des Substrats vor UV-Strahlung und schützen Oberflächen dauerhaft vor photochemischem Abbau. Sie können daher überall dort eingesetzt werden, wo ein UV-labiles Substrat vor UV-Strahlung, in erster Linie aus dem Sonnenlicht oder aus einer künstlichen Strahlenquelle, geschützt werden soll. Viele Kunststoffe, aber auch Naturstoffe wie Holz, können durch die erfindungsgemäßen Beschichtungen dauerhaft vor photochemischem Abbau geschützt werden. Die Beschichtung von Glas, die ebenfalls möglich ist, dient hingegen nicht dem Schutz des Substrats, sondern der Abschirmung von langwelliger UV-Strahlung (≥300 nm), welche z.B. handelsübliches Fensterglas nahezu vollständig durchdringt.

Gegenstand der vorliegenden Erfindung ist daher auch eine Beschichtung hergestellt aus einer erfindungsgemäßen Beschichtungszusammensetzung.

Die erfindungsgemäßen Beschichtungen weisen in bevorzugten Ausführungsformen Schichtdicken von 0,1 µm bis 20 µm, besonders bevorzugt von 0,3 µm bis 10 µm, ganz besonders bevorzugt von 0,5 µm bis 5 µm auf. Je höher der Anteil der erfindungsgemäßen Verbindungen der allgemeinen Formel (I) in diesen erfindungsgemäßen Beschichtungen ist, desto dünner können die Schichtdicken gewählt werden, um ausreichenden UV-Schutz zu erzielen.

Die erfindungsgemäßen Beschichtungen weisen bevorzugt einen Extinktionswert bei 340 nm - auch als optische Dichte bei 340 nm bezeichnet - von 1,2 oder größer, bevorzugt von 1,5 oder größer, besonders bevorzugt von 2 oder größer auf. Dieser Extinktionswert ist ein Maß für die Schutzwirkung der erfindungsgemäßen Beschichtung vor UV-Strahlung. Der Extinktionswert bei 340 nm wird mit einem UV-VIS-Spektometer Cary 50 - Varian Inc., USA nach der in den Beispielen genannten Methode A gemessen.

Weiterhin Gegenstand der vorliegenden Erfindung sind daher auch Artikel, dadurch gekennzeichnet, dass sie wenigstens ein Substrat und wenigstens eine Beschichtung hergestellt aus wenigstens einer erfindungsgemäßen Beschichtungszusammensetzung aufweisen. Als Substrate kommen dabei solche aus Kunststoff, Glas oder Naturstoffen, wie z.B. Holz, in Frage. Bevorzugt sind Substrate aus Kunststoff. Besonders bevorzugt handelt es sich bei dem Substrat um einen Formkörper, ein Extrudat oder ein Coextrudat enthaltend einen oder mehrere thermoplastische Kunststoffe. Ganz besonders bevorzugte erfindungsgemäße Artikel sind beispielsweise Folien, Platten, Stegplatten, Scheinwerferabdeckscheiben, Automobilverscheibungen oder Architekturverscheibungen.

Die erfindungsgemäßen Beschichtungszusammensetzungen eignen sich demnach erfindungsgemäß zur Beschichtung von Oberflächen, wie z.B. Kunststoffen, Holz oder Glas, insbesondere von Kunststoffoberflächen. Aufgrund ihrer hohen Transparenz können die erfindungsgemäßen Beschichtungen insbesondere auch auf transparenten Kunststoffen, vorzugsweise transparenten Thermoplasten wie Polycarbonat, Polyacrylat oder Poly(meth)acrylat, Polysulfone, Polyester, thermoplastisches Polyurethan und Polystyrol sowie deren Copolymere und Mischungen (Blends) eingesetzt werden. Geeignete Thermoplasten sind beispielsweise Polyacrylate, Poly(meth)acrylate (z.B. PMMA; z.B. Plexiglas® von der Fa. Röhm), Cycloolefin-Copolymere (COC; z.B. Topas® von der Fa. Ticona; Zenoex® von der Fa. Nippon Zeon oder Apel® von der Fa. Japan Synthetic Rubber), Polysulfone (Ultrason@ von der BASF oder Udel® von der Fa. Solvay), Polyester, wie z.B. PET oder PEN, Polycarbonat (PC), Polycarbonat/Polyester-Blends, z.B. PC/PET, Polycarbonat/Polycyclohexylmethanolcyclo-hexandicarboxylat (PCCD; Xylecs® von der Fa GE), Polycarbonat/PBT und Mischungen daraus. In besonders vorteilhafter Weise werden Polycarbonate und Copolycarbonate, vor allem Bisphenol-A-basierte (aromatische) Polycarbonate und Copolycarbonate, durch solche erfindungsgemäßen Beschichtungen vor UV-Strahlung geschützt. Derart dauerhaft vor UV-Strahlung geschütztes Polycarbonat kann dann beispielsweise für die Verglasung von Gebäuden und Fahrzeugen eingesetzt werden, wo über lange Zeiträume eine Vergilbung verhindert werden muss. Bevorzugt werden Poly(meth)acrylate sowie Polycarbonate oder Copolycarbonate eingesetzt, und insbesondere werden Polycarbonate oder Copolycarbonate und deren Mischungen eingesetzt.

Geeignete Polycarbonate für die Herstellung der erfindungsgemäßen Kunststoffzusammensetzung sind alle bekannten Polycarbonate. Dies sind Homopolycarbonate, Copolycarbonate und thermoplastische Polyestercarbonate. Die geeigneten Polycarbonate haben bevorzugt mittlere Molekulargewichte M̅_{w} von 18.000 bis 40.000, vorzugsweise von 26.000 bis 36.000 und insbesondere von 28.000 bis 35.000, ermittelt durch Messung der relativen Lösungsviskosität in Dichlormethan oder in Mischungen gleicher Gewichtsmengen Phenol/o-Dichlorbenzol geeicht durch Lichtstreuung.

Die Herstellung der Polycarbonate erfolgt vorzugsweise nach dem Phasengrenzflächenverfahren oder dem Schmelze-Umesterungsverfahren, welche mannigfaltig in der Literatur beschrieben werden. Zum Phasengrenzflächenverfahren sei beispielhaft auf H. Schnell, Chemistry and Physics of Polycarbonates, Polymer Reviews, Vol. 9, Interscience Publishers, New York 1964 S. 33 ff., auf Polymer Reviews, Vol. 10, "Condensation Polymers by Interfacial and Solution Methods", Paul W. Morgan, Interscience Publishers, New York 1965, Kap. VIII, S. 325, auf Dres. U. Grigo, K. Kircher und P. R- Müller "Polycarbonate" in Becker/Braun, Kunststoff-Handbuch, Band 3/1, Polycarbonate, Polyacetale, Polyester, Celluloseester, Carl Hanser Verlag München, Wien 1992, S. 118-145 sowie auf EP-A 0 517 044 verwiesen. Das Schmelze-Umesterungsverfahren ist beispielsweise in der Encyclopedia of Polymer Science, Vol. 10 (1969), Chemistry and Physics of Polycarbonates, Polymer Reviews, H. Schnell, Vol. 9, John Wiley and Sons, Inc. (1964) sowie in den Patentschriften DE-B 10 31 512 und US-B 6 228 973 beschrieben.

Die Polycarbonate werden aus Reaktionen von Bisphenolverbindungen mit Kohlensäureverbindungen, insbesondere Phosgen oder beim Schmelzeumesterungsprozess Diphenylcarbonat bzw. Dimethylcarbonat, erhalten. Hierbei sind Homopolycarbonate auf Basis Bisphenol-A und Copolycarbonate auf der Basis der Monomere Bisphenol-A und 1,1-Bis-(4-hydroxyphenyl)-3,3,5-trimethylcyclohexan besonders bevorzugt. Weitere Bisphenolverbindungen, die sich für die Polycarbonatsynthese einsetzen lassen, sind unter anderem offenbart in WO-A 2008037364, EP-A 1 582 549, WO-A 2002026862, WO-A 2005113639

Die Polycarbonate können linear oder verzweigt sein. Es könne auch Mischungen aus verzweigten und unverzweigten Polycarbonaten eingesetzt werden.

Geeignete Verzweiger für Polycarbonate sind aus der Literatur bekannt und beispielsweise beschrieben in den Patentschriften US-B 4 185 009, DE-A 25 00 092, DE-A 42 40 313, DE-A 19 943 642, US-B 5 367 044 sowie in hierin zitierter Literatur. Darüber hinaus können die verwendeten Polycarbonate auch intrinsisch verzweigt sein, wobei hier kein Verzweiger im Rahmen der Polycarbonatherstellung zugegeben wird. Ein Beispiel für intrinsische Verzweigungen sind so genannte Fries-Strukturen, wie sie für Schmelzepolycarbonate in der EP-A 1 506 249 offenbart sind.

Zudem können bei der Polycarbonat-Herstellung Kettenabbrecher eingesetzt werden. Als Kettenabbrecher werden bevorzugt Phenole wie Phenol, Alkylphenole wie Kresol und 4-tert.-Butylphenol, Chlorphenol, Bromphenol, Cumylphenol oder deren Mischungen verwendet.

Die Kunststoffzusammensetzung(en) der Substratschicht bzw. der Substratschichten können zusätzlich Additive, wie beispielsweise UV-Absorber, IR-Absorber sowie andere übliche Verarbeitungshilfsmittel, insbesondere Entformungsmittel und Fließmittel, sowie die üblichen Stabilisatoren, insbesondere Thermostabilisatoren sowie Antistatika, Pigmente, Farbmittel und optische Aufheller enthalten. In jeder Schicht können dabei unterschiedliche Additive bzw. Konzentrationen von Additiven vorhanden sein. Erfindungsgemäß sind jedoch zusätzliche UV-Absorber in den zu beschichtenden Kunststoffen nicht zwingend erforderlich und bevorzugt auch nicht enthalten.

Es kann aber von Vorteil sein, Kunststoffzusammensetzung(en) für die Substratschicht(en) einzusetzen, die geringe Mengen zusätzlicher UV-Absorber enthalten. In einer bevorzugten Ausführungsform wird daher als Kunststoff Polycarbonat eingesetzt, welches zusätzlich 0,01 bis 0,5 Gew.-% eines oder mehrerer UV-Absorber aus den Klassen Benzotriazol-Derivate, dimere Benzotriazol-Derivate, Triazin-Derivate, Dimere Triazin-Derivate, Diarylcyanoacrylate enthält. Dabei handelt es sich bevorzugt um solche UV-Absorber, die von den erfindungsgemäßen Verbindungen der allgemeinen Formel (I) verschieden sind.

In einer weiteren Ausführungsform können in weiteren Substratschichten, z.B. in gegebenenfalls vorhandenen Koextrusionsschichten, höhere Mengen an Additiven, insbesondere an UV-Absorbern, enthalten sein.

Im Falle von zu beschichtenden Substraten aus thermoplastischen Kunststoffen können vor allem extrudierte, coextrudierte oder spritzgegossene Formkörper beschichtet werden, beispielsweise in Form von Folien, Platten, Stegplatten sowie überwiegend flächigen Substraten. Anwendungsbereiche finden sich auch im Bereich von 1K- bzw. 2K-Spritzgussteilen, beispielsweise in Form von Scheinwerferabdeckscheiben, von Architektur- und Automobilverscheibungen.

Je nach Anwendung werden die Beschichtungen zweckmäßiger Weise auf einer oder mehreren Seiten der Substrate aufgebracht. Flächenförmige Substrate wie Folien oder Platten können dementsprechend einseitig oder zweiseitig beschichtet sein, wobei bei zweiseitiger Beschichtung die Beschichtungen gleich oder verschieden sein können.

Der Artikel enthaltend das beschichtete Substrat kann außerdem weitere Beschichtungen enthalten. Neben den UV-Schutzbeschichtungen kommen als weitere Beschichtungen beispielsweise IRabsorbierende Schichten, IR-reflektierende Schichten, elektrisch leitfähige Schichten, elektroluminisizierende Schichten, Farb- und Druckschichten zu Dekorationszwecken, elektrisch leitfähige Druckschichten, wie sie z.B. für Automobilscheibenheizung verwendet werden, gegebenenfalls auch Heizdrähte enthaltende Schichten, Antireflexionsschichten, no-drop-Beschichtungen, anti-fog-Beschichtungen, anti-fingerprint-Beschichtungen und/oder Kombinationen davon in Betracht. Diese Beschichtungen können als Zwischenschichten und/oder Außenschichten aufgebracht bzw. enthalten sein.

Zur Verbesserung der Haftung zum Substrat oder zu einer weiteren Überlackierung ist es möglich, einen geeigneten Haftvermittler einzusetzen, der für eine gute Haftung der erfindungsgemäßen Beschichtungen zum Substrat oder zu den weiteren Schichten sorgt. Der Haftvermittler kann der erfindungsgemäßen Beschichtungszusammensetzung zugesetzt werden oder wird als separate Beschichtung über der erfindungsgemäßen Schicht aufgebracht. Gängige Haftvermittler basieren meist auf Polymethacrylaten oder Polyurethanen. Neben der haftvermittelnden Wirkung kann optional durch zusätzliche UV-Absorber und weitere Lichtstabilisatoren, bevorzugt HALS, der UV-Schutz des Gesamtaufbaus erhöht werden. Die Haftvermittler bzw. Primer können wahlweise nach dem Ablüften an Raumtemperatur bei erhöhter Temperatur eingebrannt werden (bake-onbake Verfahren) oder direkt mit der Sol-Gel Lösung, bzw mit einem anderen Decklack überbeschichtet werden (wet-on-wet Verfahren).

Überdies können die aus den erfindungsgemäßen Mischungen erhaltenen Beschichtungen mit weiteren Beschichtungen überlackiert werden, was beispielsweise der Verbesserung der mechanischen Eigenschaften (Kratzfestigkeit) dienen kann. Ebenfalls ist es möglich, eine Plasmaschicht aufzubringen, die einen zusätzlichen Barriere- und Kratzschutz bieten kann. Diese Plasmaschicht wird durch Abscheidung reaktiver Spezies nach Stand der Technik appliziert - beispielsweise Plasma enhanced chemical vapor deposition PECVD, Magnetronsputtern (z.B. US-A 2007/104956) oder Aufdampfen. Derartige Beschichtungsverfahren sind bekannt und detailliert in Rene A. Haefer "Oberflächen- und Dünnschicht-Technologie" Teil I Springer-Verlag 1987 beschrieben. Typischerweise könnten hierbei glasartige Schichten abgeschieden werden. Es eignen sich aber auch diamantartige und amorphe Kohlenstoffschichten.

Insbesondere eignen sich für die Kratzfestbeschichtung Sol-Gel-Lacke. Sol-Gel-Lacke im Sinne der vorliegenden Erfindung sind Lacke, die nach dem Sol-Gel-Prozess hergestellt werden. Der Sol-Gel-Prozess ist ein Verfahren zur Synthese nichtmetallischer anorganischer oder hybridpolymerer Materialien aus kolloidalen Dispersionen, den sogenannten Solen.

Beispielsweise können solche Sol-Gel-Lacke durch Hydrolyse wässriger Dispersionen von kolloidem Siliziumdioxid und einem Organoalkoxysilan oder Mischungen aus Organoalkoxysilanen der allgemeinen Formel RSi(OR')₃ herstellt werden, wobei in den Organoalkoxysilan(en) der allgemeinen Formel RSi(OR')₃ R für einen monovalenten C₁-C₆-AlkylRest oder für einen ganz oder teilweise fluorierten C₁-C₆-Alkylrest, für eine Vinyl- oder eine Allyl-Einheit, einen Arylrest oder für einen C₁-C₆-Alkoxy-Rest steht. Besonders bevorzugt ist R ein C₁-C₄-Alkyl-Rest, beispielsweise ein Methyl-, Ethyl-, n-Propyl-, iso-Propyl-, tert.-Butyl-, sek.-Butyl- oder n-Butylrest, ein Vinyl-, Allyl-, Phenyl- oder substituierter Phenylrest. Die Reste -OR' sind unabhängig voneinander ausgewählt aus der Gruppe enthaltend C₁-C₆-Alkoxygruppen, eine Hydroxygruppe, eine Formylgruppe und eine Acetylgruppe.

Das kolloide Siliziumdioxid ist beispielsweise als z.B. Levasil^{®} 200 A, Nalco^{®} 1034A (Nalco Chemical Co), Ludox^{®} AS-40 oder Ludox^{®} LS (GRACE Davison) erhältlich. Als Organoalkoxysilane seien beispielhaft folgende Verbindungen genannt: 3,3,3-Trifluoropropytrimethoxysilan, Methyltrimethoxysilan, Methyltrihydroxysilan, Methyltriethoxysilan, Ethyltrimethoxysilan, Methyltriacetoxysilan, Ethyltriethoxysilan, Phenyltrialkoxysilan (z.B. Phenyltriethoxysilan und Phenyltrimethoxysilan) und Mischungen daraus.

Als Katalysatoren für die Hydrolyse können beispielsweise organische und/oder anorganische Säuren oder Basen verwendet werden.

In einer Ausführungsform der Herstellung von Sol-Gel-Lacken können die kolloiden Siliziumdioxidpartikel auch durch Vorkondensation ausgehend von Alkoxysilanen in situ gebildet werden (siehe hierzu "The Chemistry of Silica", Ralph K. Iler, John Wiley & Sons, (1979), p. 312-461.

Die Hydrolyse der Sol-Gel-Lösung wird durch Zugabe von Lösungsmitteln, bevorzugt alkoholischen Lösungsmitteln wie z.B. Isopropanol, n-Butanol, Isobutanol oder Mischungen daraus, abgebrochen bzw. stark verlangsamt. Anschließend können - beispielsweise zum UV-Schutz der Sol-Gel-Beschichtung - ein bzw. mehrere UV-Absorber, die optional in einem Lösungsmittel vorgelöst sind, zu der Sol-Gel Beschichtungslösung gegeben werden, wonach ein Alterungsschritt von wenigen Stunden oder mehreren Tagen/Wochen eintritt. Des Weiteren können noch weitere Additive und/oder Stabilisatoren wie beispielsweise Verlaufsmittel, Oberflächenadditive, Verdickungsmittel, Pigmente, Farbstoffe, Härtungskatalysatoren, IR-Absorber und/oder Haftvermittler zugesetzt werden.

Auch die Verwendung von Hexamethyl-disilazan oder vergleichbaren Verbindungen, die zu einer reduzierten Rissanfälligkeit der Beschichtungen führen können, ist möglich (vgl. auch WO 2008/109072 A).

Als UV-Absorber für die Sol-Gel_Beschichtungen können wahlweise z.B. die kommerziell erhältlichen mäßig polaren, meist hydroxy-haltigen UV-Absorber und/oder anorganische UV-Absorber, wie Titandioxid, Zinkoxid oder Cerdioxid, verwendet, die jedoch nicht die optimale Wirkung besitzen (EP-A 0 931 820). Mit einer Alkoxy-silyl(alkyl)-Gruppe modifizierte UV-Absorber für solche Lacksysteme auf der Basis von Resorcinol wurden in US-A 5,391,795 und US-A 5,679,820 offenbart.

Kommerziell erhältliche UV stabilisierte Sol-Gel Lacke sind beispielsweise von der Fa. Momentive Performance Materials unter den Produktbezeichnungen AS4000 und AS4700 erhältlich. Sol-Gel Siloxanlacke besitzen bei Schichtdicken von 1 bis 20µm, bevorzugt 2 bis 15 µm, besonders bevorzugt 4 bis 12 µm eine Extinktion zwischen 0,2 und 4, bevorzugt 0,2 und 2, besonders bevorzugt 0,3 ≤ Extinktion (Sol-Gel-Schicht) ≤1,5.

Die erfindungsgemäße Beschichtung eignet sich besonders bevorzugt als Primerschicht für kratzfeste Beschichtungen, die mittels Sol-Gel-Lacken aufgebracht werden. Letztere zeigen eine ausgezeichnete Haftung auf den erfindungsgemäßen Beschichtungen. Hierzu ist vorzugsweise keine zusätzliche Haftvermittlerschicht erforderlich.

Hybridpolymere Lacke im Sinne der vorliegenden Erfindung - auch Hybridlacke genannt - basieren auf der Verwendung von Hybridpolymeren als Bindemittel. Hybridpolymere (Hybride: lat. "von zweierlei Herkunft") sind polymere Werkstoffe, die Struktureinheiten verschiedener Materialklassen auf molekularer Ebene in sich vereinen. Durch ihren Aufbau können Hybridpolymere völlig neuartigen Eigenschaftskombinationen aufweisen. Im Unterschied zu Verbundwerkstoffen (definierte Phasengrenzen, schwache Wechselwirkungen zwischen den Phasen) und Nanokompositen (Verwendung nanoskaliger Füllstoffe) sind die Struktureinheiten von Hybridpolymeren auf molekularer Ebene miteinander verknüpft. Dies gelingt durch chemische Verfahren wie z. B. den Sol-Gel-Prozess, mit dem anorganische Netzwerke aufgebaut werden können. Durch den Einsatz von organisch reaktiven Precursoren z. B. organisch modifizierten Metall-Alkoxiden können zusätzlich organische Oligomer/Polymerstrukturen erzeugt werden. Oberflächenmodifizierte Nanopartikel enthaltende Acrylatlacke, die nach der Härtung ein organisch/anorganisches Netzwerk bilden, werden ebenfalls als Hybridlack definiert.

Ein möglicher thermisch härtbarer Hybridlack ist der PHC587B oder PHC587C (Momentive Performance Materials) siehe auch EP-A 0 570 165. Die Schichtdicke sollte zwischen 1 bis 20 µm, bevorzugt 3 bis 15 µm besonders bevorzugt 6 bis 8 µm betragen.

UV härtbare Hybridlacke sind beispielsweise UV-härtbare Acrylatlacke oder UV-härtbare wasserfreie hydrolisierbare Silan-Systeme, wie sie in WO 2008/071363 A oder DE-A 2804283 beschrieben werden. Ein kommerziell erhältliches System ist der UVHC3000 (Momentive Performance Materials). Die Schichtdicke sollte zwischen 1 bis 25 µm, bevorzugt 4 bis 20 µm besonders bevorzugt 8 bis 12 µm betragen. Die Kratzschutzschichten auf Basis von Hybridlacken sollten eine Extinktion bei 340 nm zwischen 0,1 und 3, bevorzugt zwischen 0,2 und 2,5, besonders bevorzugt 0,3 ≤ Extinktion (Hybridschicht) ≤ 2 besitzen.

Die folgenden Ausführungsbeispiele diesen der exemplarischen Erläuterung der Erfindung, ohne diese jedoch einzuschränken.

### Beispiele

### Methode A: Extinktionsbestimmung (Bestimmung der optischen Dichte) bei 340 nm

Die Extinktion der erfindungsgemäßen Beschichtung nach Applikation auf ein Polycarbonatsubstrat (oder ein Glassubstrat für Methode B) und anschließender Härtung wurde mit Hilfe eines Cary 50 UV-Vis Spektrophotometers von Varian Inc., USA bei 340 nm bestimmt, wobei ein unbeschichtetes, aber sonst identisches Polycarbonatsubstrat (Glassubstrat für Methode B) als Hintergrundspektrum verwendet wurde.

### Methode B: Schichtdickenbestimmung

Für die Bestimmung der Schichtdicke der Beschichtungen auf den polymeren Substraten wurden zur Kalibrierung die betreffenden Lackzusammensetzung jeweils in unterschiedlicher Schichtdicke auf Glas aufgetragen und gehärtet, die Extinktion der jeweiligen Beschichtung bei 340 nm gemäß Methode A bestimmt und die Schichtdicken der Beschichtungen mit Hilfe eines Profilmessers Alpha-Step 500 (Fa. Tencor) bestimmt. Anschließend wurde anhand der mit Methode A) ermittelten Extinktion bei 340 nm der betreffenden Beschichtung auf dem Polycarbonatsubstrat, deren Schichtdicke bestimmt werden sollte, die Schichtdicke dieser Beschichtung berechnet (Extinktion der jeweiligen Beschichtung bei 340 nm ist proportional der Schichtdicke dieser Beschichtung).

### Beispiel 1: Herstellung des erfindungsgemäßen UV-Absorbers

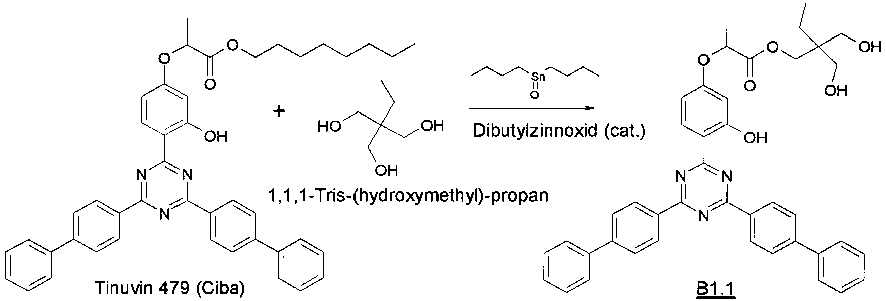

110 g Tinuvin 479 (Ciba Specialty Chemicals Inc., Schweiz) und 8,1 g Dibutylzinnoxid (Aldrich) wurden in 217,7 g 1,1,1-Tris-(hydroxymethyl)-propan (Aldrich) vorgelegt und 5 h bei 165°C (Temperatur des Ölbads) gerührt. Die Rührmasse war vorerst trübe, dann wurde sie klar. Nach dem Abkühlen des Reaktionsgemisches wurde das Produkt mit Methanol kristallin ausgefällt, abfiltriert, mit Methanol gespült und getrocknet. Die weitere Reinigung erfolgte durch zweimalige Kristallisation aus Toluol. Der Schmelzpunkt von **B1.1** beträgt 121,8°C. Ausbeute: 70 g (63% der Theorie)
Elementaranalyse: C₄₂H₃₉N₃O₆ (681,80)
Ber.: C73,99; H5,77; N6,16.
Gef.: C74,80; H6,00; N5,90. 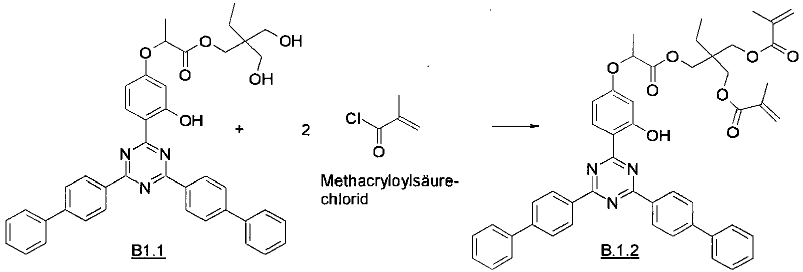 70 g **B1.1** wurden in 350 ml Dioxan gelöst und 31,2 g Triethylamin zugegeben. Dieser Lösung wurde bei äußerer Kühlung tropfenweise eine Lösung aus 23 g Methacryloylsäurechlorid in 70 ml Dioxan zugegeben. Das Reaktionsgemisch wurd 4 h bei Raumtemperatur gerührt und anschließend in Wasser gegeben. Die abgesetzte Substanz wurde in eine Lösung aus Toluol und Ethylacetat (8:1) aufgenommen. Die Lösung wurd mit Magnesiumsulfat getrocknet. Die anschließende chromatographische Reinigung erfolgte auf Kieselgel in Toluol/Ethylacetat(8:1). Nach dem Entfernen des Lösungsmittels wurde die Verbindung **B.1.2**, im Vakuum getrocknet. Das Produkt war ein nicht kristallines glasartiges festes Harz. Ausbeute: 29 g (35% der Theorie.)
Elementaranalyse: C₅₀H₄₇N₃O₈ (817,95)
Ber.: C73,42; H5,79; N5,14.
Gef.: C73,20; H6,00; N5,10.

### Beispiel 2: Herstellung der erfindungsgemäßen Lackformulierungen (Beschichtungszusammensetzungen)

a) Zu 9 g Diacetonalkohol wurde 1 g **B.1.2** zugegeben und bei Raumtemperatur restlos gelöst. 0,02 g Darocur^{®} 4265 wurden zugegeben. Die klare Lösung wurde über eine Drucknutsche filtriert (Membranfilter mit der Porengröße 0,2 µm) und in eine Flasche aus Dunkelglas überführt.
b) Zu 9 g Diacetonalkohol wurden 0,9 g **B.1.2** und 0,1 g Dipentaerythritol penta/hexa-acrylate (DPHA, Fa Aldrich) zugegeben und bei Raumtemperatur restlos gelöst. 0,02 g Darocur^{®} 4265 wurden zugegeben. Die klare Lösung wurde über eine Drucknutsche filtriert (Membranfilter mit der Porengröße 0,2 µm) und in eine Flasche aus Dunkelglas überführt.
c) Analog zu b) wurde aus 0,8 g **B.1.2** und 0,2 g Dipentaerythritol penta/hexa-acrylate (DPHA, Fa Aldrich) eine weitere Lackformulierung hergestellt
d) Analog zu b) wurde aus 0, 5 g **B.1.2** und 0, 5 g Dipentaerythritol penta/hexa-acrylate (DPHA, Fa Aldrich) eine weitere Lackformulierung hergestellt.
e) Analog zu b) wurde aus 0,25 g **B.1.2** und 0,75 g Dipentaerythritol penta/hexa-acrylate (DPHA, Fa Aldrich) eine weitere Lackformulierung hergestellt.
f) Zu 6,13 g Diacetonalkohol wurden 0,5 g **B.1.2** zugegeben und bei Raumtemperatur restlos gelöst. 3,33 g des Lackes UVHC 3000 (Fa.Momentive Performance Materials) und 0,04 g Darocur^{®} 4265 wurden zugegeben. Die klare Lösung wurde über eine Drucknutsche filtriert (Membranfilter mit der Porengröße 0,2 µm) und in eine Flasche aus Dunkelglas überführt.

### Beispiel 3: Herstellung der beschichteten Polycarbonatgegenstände

### 3.1. Substrate und deren Vorbereitung:

Spritzgegossene Polycarbonat (PC)-Platten in optischer Qualität aus Makrolon^{®} M2808 (Bayer MaterialScience AG; mittelviskoses Bisphenol A-Polycarbonat, MVR 10g/10min nach ISO 1133 bei 300°C und 1,2 kg, ohne UV-Stabilisierung) der Größe 10 x 15 x 0,32cm wurden 1 h bei 120°C getempert, mit Isopropanol gespült und abgelüftet.

Analog wurden spritzgegossene Polycarbonat (PC)-Platten gleicher Größe in optischer Qualität aus Makrolon^{®} AL2647 (mittelviskoses Bisphenol A-Polycarbonat mit UV-Stabilisator und Formtrennmittel; MFR 13 g/10min nach ISO 1133 bei 300°C und 1,2 kg) präpariert.

Einer beidseitig mit Kaschierfolien bestückten Polycarbonat (PC)-Folie (Makrofol^{®} DE 1-1 cc, Dicke 500µm) wurden beidseitig die Kaschierfolien abgenommen. Die Folien wurden ohne nasse Reinigung und ohne thermische Vorbehandlung beschichtet.

### 3.2. Applikation der erfindungsgemäßen UV-Schutzschicht

a) Die Flüssige Lackformulierung aus Beispiel 2a) wurde auf die Platten oder auf die Folien mit Hilfe des Filmziehgerätes Zehntner ZAA 2300 (Spiralrakel 9 µm, Ziehgeschwindigkeit 15 mm/s) appliziert. Die Beschichtungen wurden kurz angetrocknet und unter der UV-Lampe ausgehärtet. Dazu wurde die UV-Anlage BK 150 EBU Fa. Arccure technologies GmbH verwendet (UV-Strahler 150 D 200, elektrische Leistung 3 kW, UV-Dosis über den gesamten Spektralbereich 15 J/cm²).
   Die Dicke der so erhaltenen Beschichtungen betrug zwischen 1 und 2 µm. Die optische Dichte wurde bei 340 nm (gemessen mit UV-VIS-Spektometer Cary 50 - Varian Inc., USA) gemessen und lag über 3. Die Haftung dieser Beschichtungen wurde durch Klebebandabriss (Klebeband 3M^{®} 610) sowie mit Gitterschnitt (analog zu ISO 2409) bestimmt. Der Test wurde bestanden, d.h. es kam zu keinerlei Abriss der Beschichtung (Bewertung 0 gemäß ISO 2409).
b) bis e) Analog zu a) wurden die Lackformulierungen 2b) bis 2e) aufgetragen und ausgehärtet. Die Dicke der so erhaltenen Beschichtungen betrug zwischen 1 und 2 µm. Die optische Dichte lag bei 340 nm über 2. Der Haftungstest wurde bestanden (Bewertung 0 gemäß ISO 2409).
f) Analog zu a) wurde die Lackformulierung 2f) aufgetragen und ausgehärtet. Die Dicke der so erhaltenen Beschichtung betrug 2,9 µm. Der Haftungstest wurde bestanden (Bewertung 0 gemäß ISO 2409). Die optische Dichte lag bei 340 nm bei 3,76.

### 3.3 Applikation der Decklackschicht:

a) Die mit der UV-Schutzschicht aus Beispiel 3.2.a) versehenen Platten, bzw. Folien wurden anschließend mit einem kommerziell erhältlichen PMMA-Primer (Primer SHP470 der Firma Momentive Performance Materials) beschichtet, 30 min bei Raumtemperatur abgelüftet und für 30 min bei 127°C gehärtet. Daran angeschlossen wurde direkt mit dem frisch filtrierten kommerziell erhältlichen Sol-Gel Lack AS4700 der Firma Momentive Performance Materials im Flutverfahren überlackiert. Nach 30 min Ablüften bei Raumtemperatur wurden die Platten bei 127°C für eine Stunde gehärtet.
   Die Haftung dieser Beschichtungen wurde durch Klebebandabriss (Klebeband 3M^{®} 610) sowie mit Gitterschnitt (analog zu ISO 2409) bestimmt. Der Test wurde bestanden, d.h. es kam zu keinerlei Abriss der Beschichtung (Bewertung 0 gemäß ISO 2409).
b) Die mit der UV-Schutzschicht aus Beispiel 3.2a) versehenen Platten, bzw. Folien wurden anschließend mit einem kommerziell erhältlichen Lack UVHC 3000 der Firma Momentive Performance Materials im Flutverfahren überlackiert. Nach 6 min Ablüften in Trockenschrank bei 70°C wurden die Platten, bzw. Folien unter der UV-Lampe ausgehärtet. Die Beschichtung bestand den Haftungstest, d.h. es kam zu keinerlei Abriss der Beschichtung (Bewertung 0 gemäß ISO 2409).
c) Die mit der UV-Schutzschicht aus Beispiel 3.2e) versehenen Platten wurden anschließend in die Vakuumkammer eingebracht und ca. 180 mm vor der Plasmaquelle befestigt. Die Vakuumkammer wurde anschließend auf einen Basisdruck kleiner 1·10⁻⁴ mbar evakuiert. Danach wurden ein Fluss nach 24g/h Hexamethylsiloxan eingestellt und das Plasma gezündet mit einer Leistung von 2000W, gepulst Tₒₙ=5 ms und T_{off}=5 ms. Die Schichtabscheidung lief für 1 min bei einem Druck von p=0.025 mbar. Danach wurde der Sauerstoff zugeschaltet und der Fluss von 0 auf 1 l/min über 30 s erhöht. Bei einem Fluss von 24 g/h HMDSO und 1 l/min O₂ wurde bei einem Druck von p=0,14 mbar für weitere 8 min bei der o.g. Leistung Schichtabscheidung betrieben. Dies ergab eine Gesamtschichtdicke von ca. 5 µm. Die Schicht war transparent und wies eine Bleistifthärte von 3H auf. Die Beschichtung bestand den Haftungstest, d.h. es kam zu keinerlei Abriss der Beschichtung (Bewertung 0 gemäß ISO 2409).

Die Beispiele zeigen, dass die erfindungsgemäßen UV-Absorber selbst filmbildende Eigenschaften aufweisen und zur Bildung einer klaren, dünnen, gut haftenden Schicht keinerlei zusätzlicher Filmbildner, wie z.B. Bindemittel oder polymerisierbarer Comonomere, bedürfen (vgl. insbesondere Beispiele mit Beschichtungsformulierung aus Beispiel 2a)). Die Zugabe von unterschiedlichen Mengen an Reaktivverdünnern in den Beispielen 2b) bis 2e) zeigt vergleichbare Ergebnisse. Die Bespiele zeigen weiterhin, dass die mit der erfindungsgemäßen Beschichtung UVgeschützten Substrate durch Plasma oder mit kommerziell erhältlichen UV-härtenden oder Sol-Gel-Rezepturen kratzfest überlackiert werden können. Zudem konnte gezeigt werden, dass die erfindungsgemäßen Beschichtungen selbst direkt als Primerschichten für die Kratzfestbeschichtungen dienen können, ohne dass hierbei die Haftung beeinträchtigt wird.

## Patentansprüche

1. Verbindungen der allgemeinen Formel (I)
A - X (- T)ₙ (I),
wobei
A für 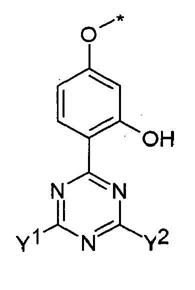 steht, worin
Y¹ und Y² unabhängig voneinander für Substituenten der generellen Formel 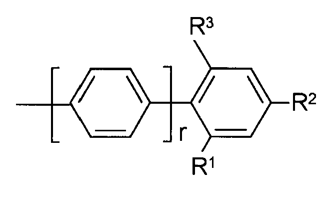 stehen, worin
r für 0 oder 1, bevorzugt für 1 steht,
R¹, R², R³ unabhängig voneinander für H, OH, C₁₋₂₀-Alkyl, C₄₋₁₂-Cycloalkyl, C₂₋₂₀-Alkenyl, C₁₋₂₀-Alkoxy, C₄₋₁₂-Cycloalkoxy, C₂₋₂₀-Alkenyloxy, C₇₋₂₀-Aralkyl, Halogen, -C≡N, C₁₋₅-Haloalkyl, -SO₂R', -SO₃H, - SO₃M (M = Alkalimetall), -COOR', -CONHR', -CONR'R", - OCOOR', -OCOR', -OCONHR', (Meth)acrylamino, (Meth)acryloxy, gegebenenfalls durch C₁₋₁₂-Alkyl, C₁₋₁₂-Alkoxy, CN und/oder Halogen substituiertes C₆₋₁₂-Aryl oder gegebenenfalls durch C₁₋₁₂-Alkyl, C₁₋₁₂-Alkoxy, CN und/oder Halogen substituiertes C₃₋₁₂-Heteroaryl stehen, worin
M für ein Alkalimetallkation steht,
R' und R" für H, C₁₋₂₀-Alkyl, C₄₋₁₂-Cycloalkyl, gegebenenfalls durch C₁₋₁₂-Alkyl, C₁₋₁₂-Alkoxy, CN und/oder Halogen substituiertes C₆₋₁₂-Aryl oder gegebenenfalls durch C₁₋₁₂-Alkyl, C₁₋₁₂-Alkoxy, CN und/oder Halogen substituiertes C₃₋₁₂-Heteroaryl stehen,
X für einen gegebenenfalls substituierten linearen oder verzweigten offenkettigen Linker aus Kohlenstoff, Sauerstoff, Stickstoff, Schwefel, Phosphor und/oder Silizium in der Kette steht,
T für einen Acrylat-Rest -O-(C=O)-CH=CH₂ oder einen Methacrylat-Rest -O-(C=O)-C(CH₃)=CH₂ steht, und
n für eine ganze Zahl von 2 bis 5 steht.

2. Verbindungen gemäß Anspruch 1, **dadurch gekennzeichnet, dass** es sich um solche der allgemeinen Formel (1-1) handelt 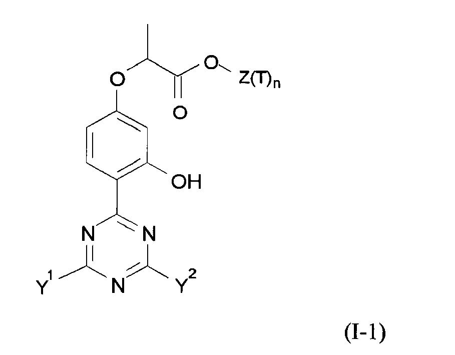 worin
Z für einen gegebenenfalls substituierten linearen oder verzweigten C₁₋₂₀-Alkylen-Rest oder C₁₋₂₀-Alkylenether-Rest steht,
T, n, Y¹ und Y² die in Anspruch 1 genannte Bedeutung haben.

3. Verbindungen gemäß Anspruch 1 oder 2, **dadurch gekennzeichnet, dass** in den Substituenten Y¹ und Y² jeweils r für 1 steht.

4. Verbindungen gemäß wenigstens einem der Ansprüche 1 bis 3, **dadurch gekennzeichnet, dass** in den Substituenten Y¹ und Y² jeweils die Reste R¹, R² und R³ für H stehen

5. Verbindungen gemäß wenigstens einem der Ansprüche 1 bis 4, **dadurch gekennzeichnet, dass** es sich um solche Verbindungen ausgewählt aus der Gruppe der Verbindungen der Formel (I-1-1) bis (I-1-12) handelt

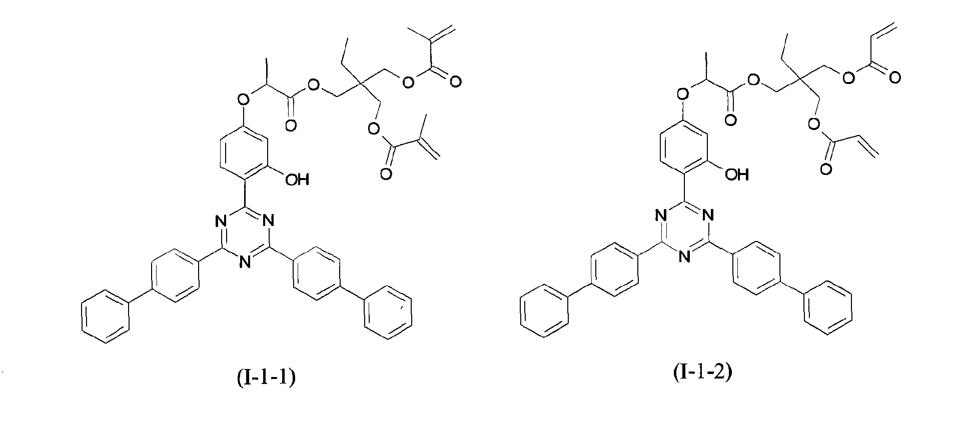

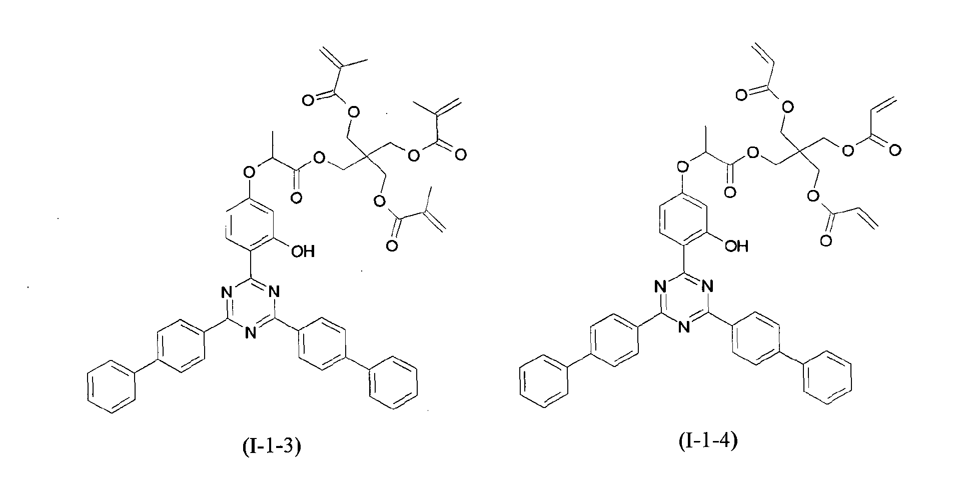

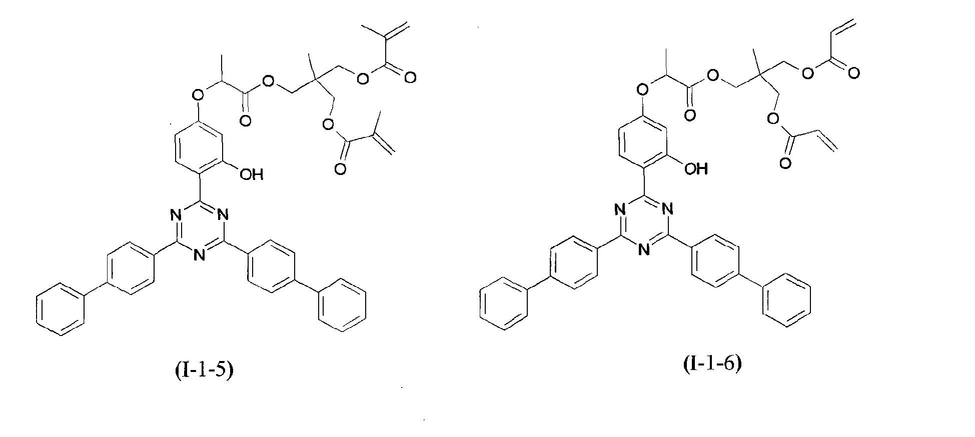

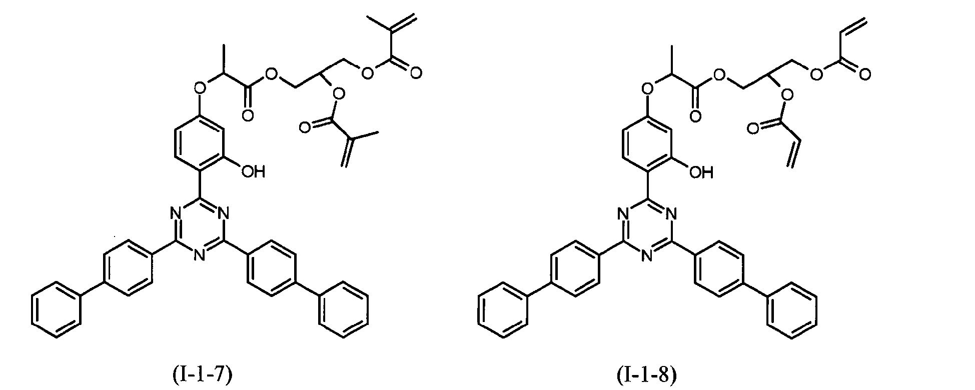

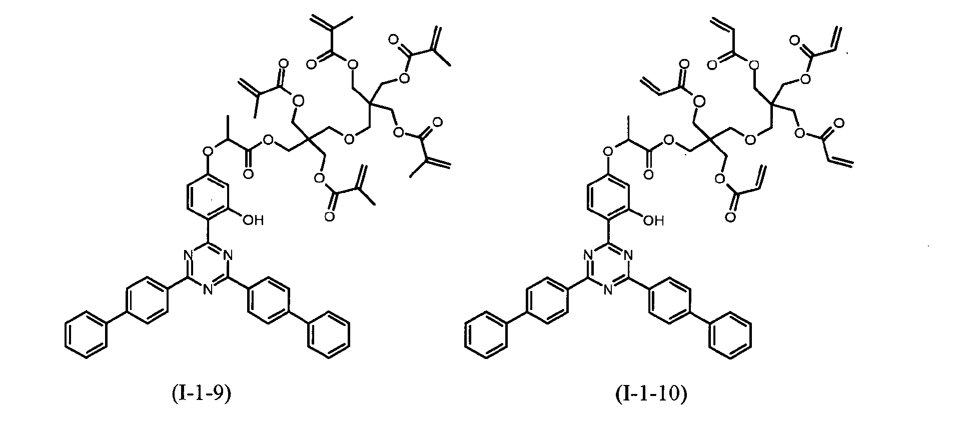

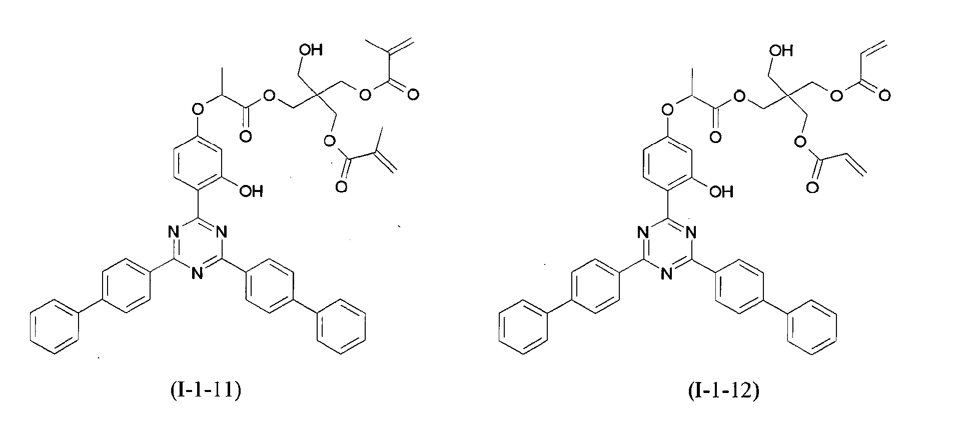

6. Beschichtungszusammensetzung, **dadurch gekennzeichnet, dass** sie wenigstens eine der Verbindungen gemäß wenigstens einem der Ansprüche 1 bis 5 und wenigstens ein organisches Lösemittel enthält.

7. Beschichtungszusammensetzung gemäß Anspruch 6, **dadurch gekennzeichnet, dass** sie wenigstens einen Initiator, bevorzugt wenigstens einen Photoinitiator enthält.

8. Beschichtungszusammensetzung gemäß Anspruch 6 oder 7, **dadurch gekennzeichnet, dass** sie wenigstens einen Stabilisator aus der Gruppe der HALS-Stabilisatoren (Hindered Amine Light Stabiliser) enthält.

9. Beschichtungszusammensetzung gemäß wenigstens einem der Ansprüche 6 bis 8, **dadurch gekennzeichnet, dass** sie wenigstens einen Reaktivverdünner oder wenigstens ein oligomeres aliphatisches Urethanacrylat oder Polyesteracrylat enthält.

10. Beschichtungszusammensetzung gemäß wenigstens einem der Ansprüche 6 bis 9, **dadurch gekennzeichnet, dass** sie zusätzlich ein oder mehrere weitere Additive ausgewählt aus der Gruppe enthaltend Stabilisatoren, Verlaufsmittel, Oberflächenadditive, Pigmente, Farbstoffe, Haftvermittler, IR-Absorber und UV-Absorber verschieden von den Verbindungen der allgemeinen Formel (I) enthält.

11. Verfahren zur Beschichtung von Substraten, **dadurch gekennzeichnet, dass**
A) auf das Substrat eine Schicht aus einer Beschichtungszusammensetzung gemäß wenigstens einem der Ansprüche 6 bis 10 aufgebracht wird,
B) anschließend wenigstens ein Teil des Lösemittels aus der gemäß Schritt A) erhaltenen Schicht entfernt wird,
C) anschließend die gemäß Schritt B) erhaltene Schicht ausgehärtet wird.

12. Beschichtung hergestellt aus einer Beschichtungszusammensetzung gemäß wenigstens einem der Ansprüche 6 bis 10.

13. Artikel, **dadurch gekennzeichnet, dass** er wenigstens ein Substrat und wenigstens eine Beschichtung hergestellt aus wenigstens einer Beschichtungszusammensetzung gemäß wenigstens einem der Ansprüche 6 bis 10 enthält.

14. Artikel gemäß Anspruch 13, **dadurch gekennzeichnet, dass** es sich bei dem Substrat um einen Formkörper, ein Extrudat oder ein Coextrudat enthaltend einen oder mehrere thermoplastische Kunststoffe handelt.

15. Folie, Platte, Stegplatte, Scheinwerferabdeckscheibe, Automobilverscheibung oder Architekturverscheibung gemäß Anspruch 13 oder 14.

## Claims

1. Compounds of the general formula (I)
A - X (- T)ₙ (I),
wherein
A represents 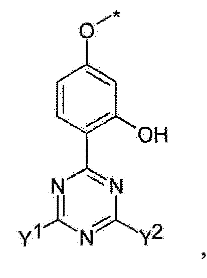 wherein
Y¹ and Y² independently of one another represent substituents of the general formula 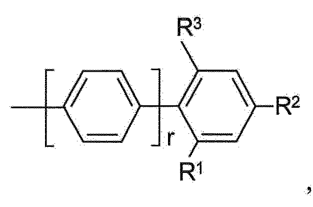 wherein
r represents 0 or 1, preferably 1,
R¹, R², R³ independently of one another represent H, OH, C₁₋₂₀-alkyl, C₄₋₁₂-cycloalkyl, C₂₋₂₀-alkenyl, C₁₋₂₀-alkoxy, C₄₋₁₂-cycloalkoxy, C₂₋₂₀-alkenyloxy, C₇₋₂₀-aralkyl, halogen, -C≡N, C₁₋₅-haloalkyl, -SO₂R', -SO₃H, -SO₃M (M = alkali metal), -COOR', -CONHR', -CONR'R", -OCOOR', -OCOR', -OCONHR', (meth)acrylamino, (meth)acryloxy; C₆₋₁₂-aryl optionally substituted by C₁₋₁₂-alkyl, C₁₋₁₂-alkoxy, CN and/or by halogen, or C₃₋₁₂-heteroaryl optionally substituted by C₁₋₁₂-alkyl, C₁₋₁₂-alkoxy, CN and/or by halogen, wherein
M represents an alkali metal cation,
R' and R" represent H, C₁₋₂₀-alkyl, C₄₋₁₂-cycloalkyl; C₆₋₁₂-aryl optionally substituted by C₁₋₁₂-alkyl, C₁₋₁₂-alkoxy, CN and/or by halogen, or C₃₋₁₂-heteroaryl optionally substituted by C₁₋₁₂-alkyl, C₁₋₁₂-alkoxy, CN and/or by halogen,
X represents an optionally substituted linear or branched open-chain linker of carbon, oxygen, nitrogen, sulfur, phosphorus and/or silicon in the chain,
T represents an acrylate radical -O-(C=O)-CH=CH₂ or a methacrylate radical -O-(C=O)-C(CH₃)=CH₂, and
n represents an integer from 2 to 5.

2. Compounds according to claim 1, **characterised in that** they are compounds of the general formula (I-1) 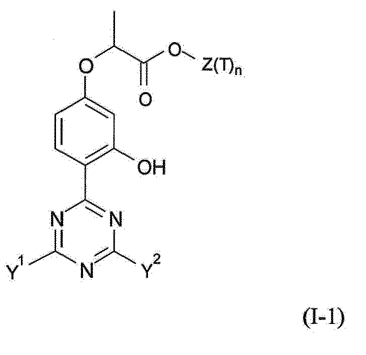 wherein
Z represents an optionally substituted linear or branched C₁₋₂₀-alkylene radical or C₁₋₂₀-alkylene ether radical,
T, n, Y¹ and Y² have the meanings given in claim 1.

3. Compounds according to claim 1 or 2, **characterised in that** in the substituents Y¹ and Y², r in each case represents 1.

4. Compounds according to at least one of claims 1 to 3, **characterised in that** in the substituents Y¹ and Y², the radicals R¹, R² and R³ in each case represent H.

5. Compounds according to at least one of claims 1 to 4, **characterised in that** they are compounds selected from the group of compounds of formulae (I-1-1) to (I-1-12)

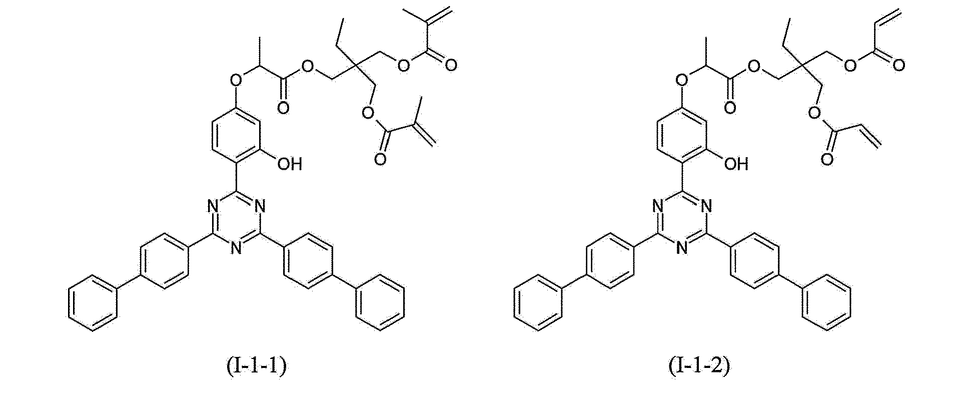

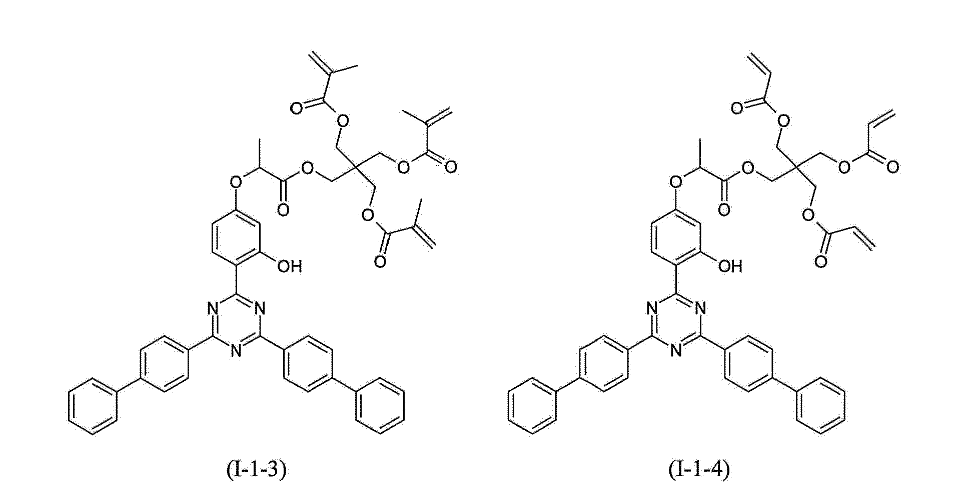

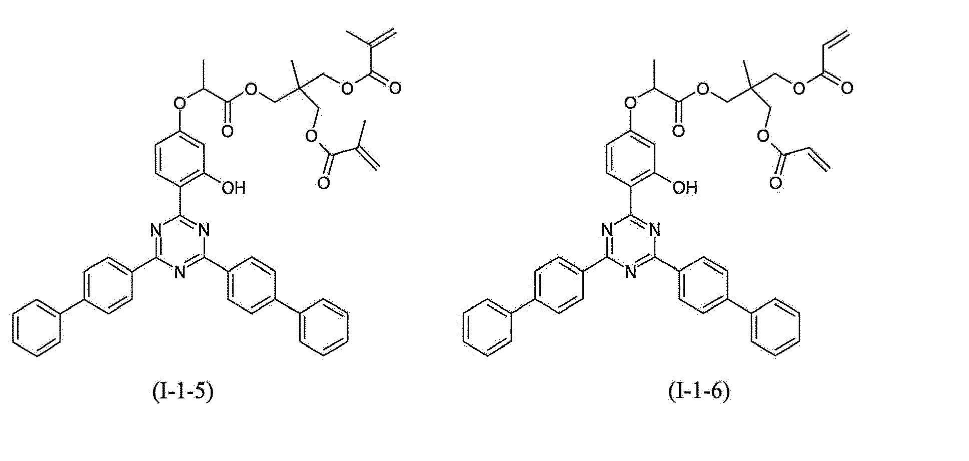

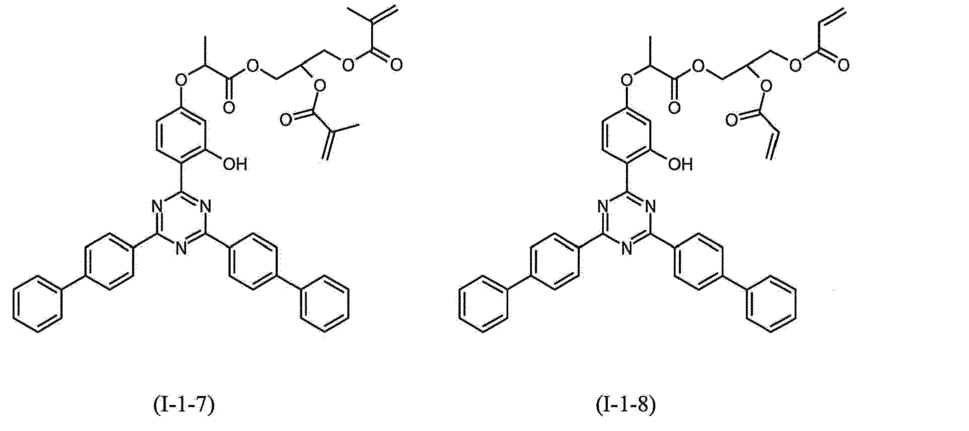

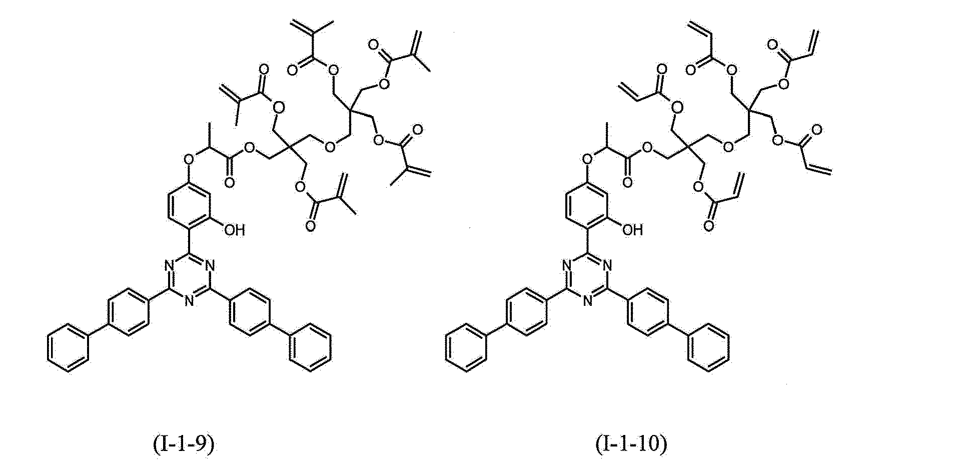

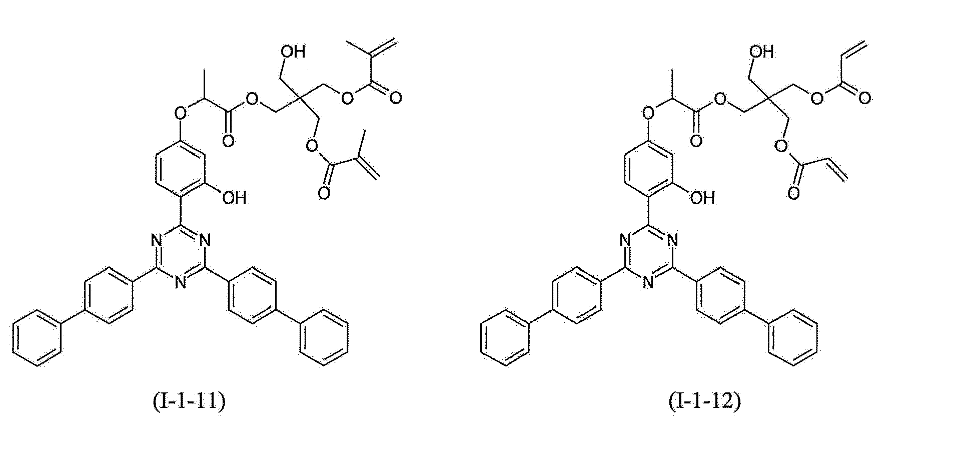

6. Coating composition, **characterised in that** it comprises at least one of the compounds according to at least one of claims 1 to 5 and at least one organic solvent.

7. Coating composition according to claim 6, **characterised in that** it comprises at least one initiator, preferably at least one photoinitiator.

8. Coating composition according to claim 6 or 7, **characterised in that** it comprises at least one stabiliser from the group of the HALS stabilisers (Hindered Amine Light Stabiliser).

9. Coating composition according to at least one of claims 6 to 8, **characterised in that** it comprises at least one reactive diluent or at least one oligomeric aliphatic urethane acrylate or polyester acrylate.

10. Coating composition according to at least one of claims 6 to 9, **characterised in that** it additionally comprises one or more further additives selected from the group containing stabilisers, flow agents, surface additives, pigments, colourants, adhesion promoters, IR absorbers, and UV absorbers other than the compounds of the general formula (I).

11. Method of coating substrates, **characterised in that**
A) a layer of a coating composition according to at least one of claims 6 to 10 is applied to the substrate,
B) then at least part of the solvent is removed from the layer obtained according to step A),
C) then the layer obtained according to step B) is cured.

12. Coating produced from a coating composition according to at least one of claims 6 to 10.

13. Article, **characterised in that** it comprises at least one substrate and at least one coating produced from at least one coating composition according to at least one of claims 6 to 10.

14. Article according to claim 13, **characterised in that** the substrate is a moulded article, an extrudate or a coextrudate comprising one or more thermoplastic plastics.

15. Film, sheet, multi-wall sheet, headlamp cover plate, automotive glazing or architectural glazing according to claim 13 or 14.

## Revendications

1. Composés de formule générale (I)
A-X(-T)ₙ (I),
dans laquelle
A représente 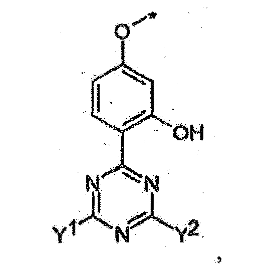
Y¹ et Y² représentant indépendamment l'un de l'autre des substituants de formule générale 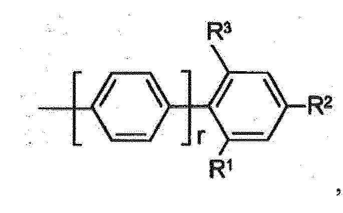 dans laquelle
r représente 0 ou 1, de préférence 1,
R¹, R², R³ représentent indépendamment les uns des autres H, OH, alkyle en C₁₋₂₀, cycloalkyle en C₄₋₁₂, alcényle en C₂₋₂₀, alcoxy en C₁₋₂₀, cycloalcoxy en C₄₋₁₂, alcényloxy en C₂₋₂₀, aralkyle en C₇₋₂₀, halogène, -C=N, haloalkyle en C₁₋₅, -SO₂R', -SO₃H, -SO₃M (M = métal alcalin), -COOR', -CONHR', - CONR'R", -OCOOR', -OCOR', -OCONHR', (méth)acrylamino, (méth)acryloxy, aryle en C₆₋₁₂ éventuellement substitué par alkyle en C₁₋₁₂, alcoxy en C₁₋₁₂, CN et/ou halogène, ou hétéroaryle en C₃₋₁₂ éventuellement substitué par alkyle en C₁₋₁₂, alcoxy en C₁₋₁₂, CN et/ou halogène,
M représentant un cation de métal alcalin,
R' et R" représentant H, alkyle en C₁₋₂₀, cycloalkyle en C₄₋₁₂, aryle en C₆₋₁₂ éventuellement substitué par alkyle en C₁₋₁₂, alcoxy en C₁₋₁₂, CN et/ou halogène, ou hétéroaryle en C₃₋₁₂ éventuellement substitué par alkyle en C₁₋₁₂, alcoxy en C₁₋₁₂, CN et/ou halogène,
X représente un lieur à chaîne ouverte, linéaire ou ramifié, éventuellement substitué, constitué de carbone, d'oxygène, d'azote, de soufre, de phosphore et/ou de silicium dans la chaîne,
T représente un radical acrylate -O-(C=O)-CH=CH₂ ou un radical méthacrylate -O-(C=O)-C(CH₃)=CH₂, et
n représente un nombre entier de 2 à 5.

2. Composés selon la revendication 1, **caractérisés en ce qu'**il s'agit de composés de formule générale (I-1) 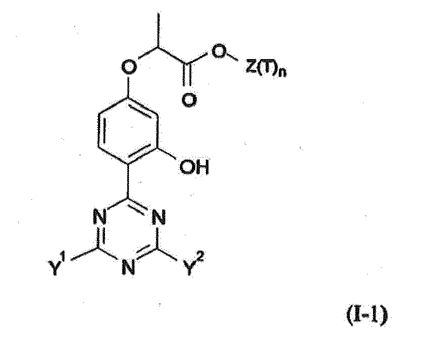 dans laquelle
Z représente un radical alkylène en C₁₋₂₀ linéaire ou ramifié, éventuellement substitué, ou un radical éther d'alkylène en C₁₋₂₀,
T, n, Y¹ et Y² ont la signification indiquée dans la revendication 1.

3. Composés selon la revendication 1 ou 2, **caractérisés en ce que** r représente à chaque fois 1 dans les substituants Y¹ et Y².

4. Composés selon au moins l'une quelconque des revendications 1 à 3, **caractérisés en ce que** les radicaux R¹, R² et R³ représentent à chaque fois H dans les substituants Y¹ et Y².

5. Composés selon au moins l'une quelconque des revendications 1 à 4, **caractérisés en ce qu'**il s'agit de composés choisis dans le groupe constitué par les composés de formule (I-1-1) à (I-1-12)

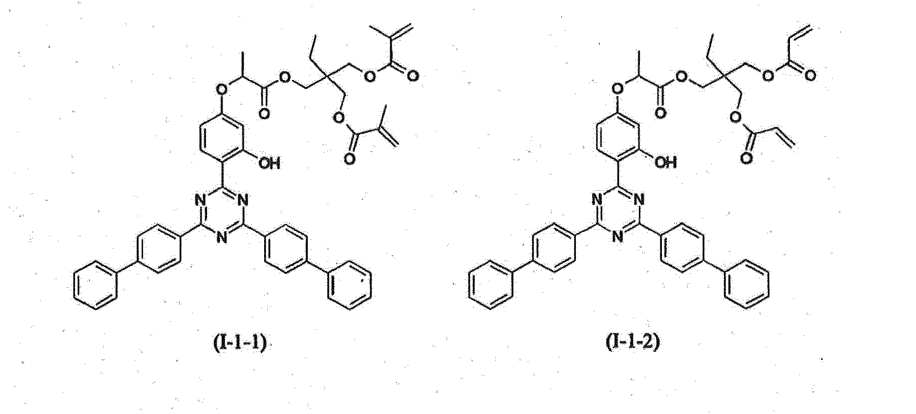

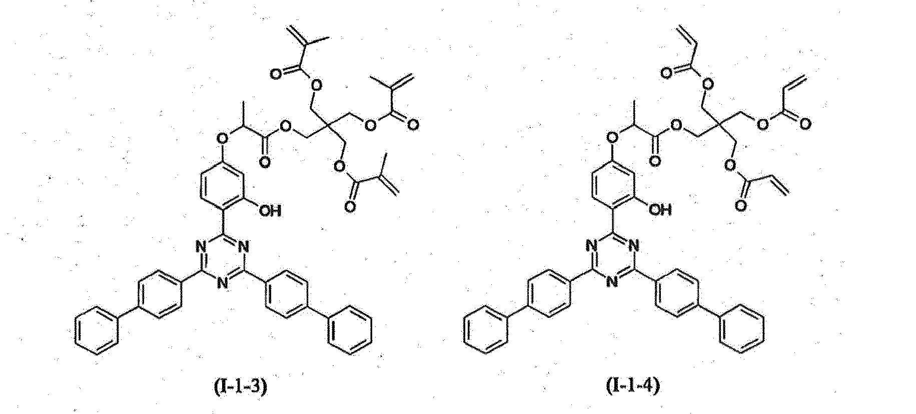

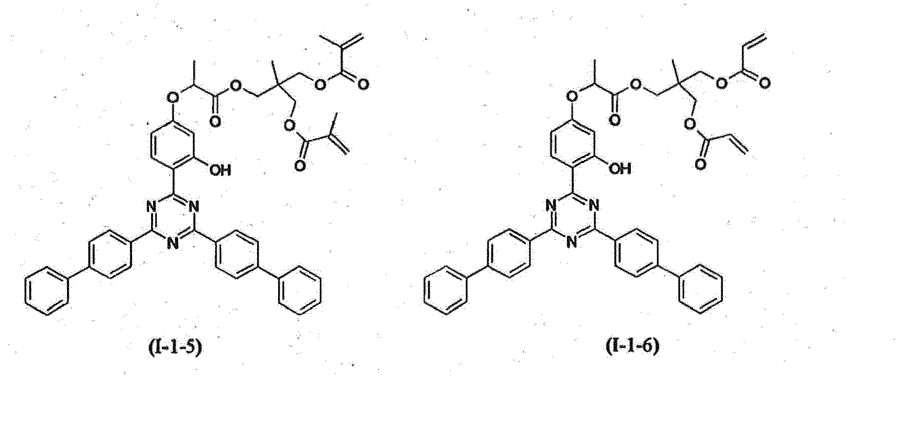

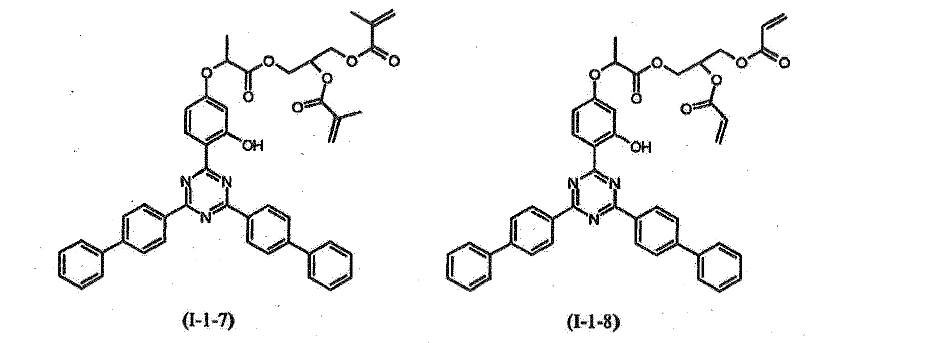

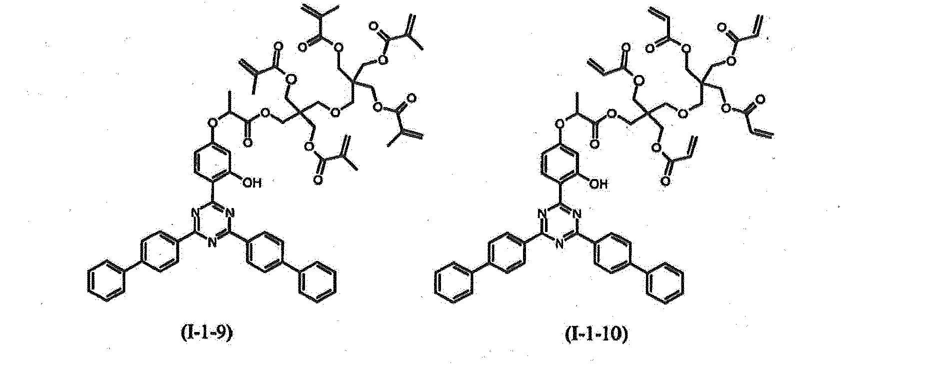

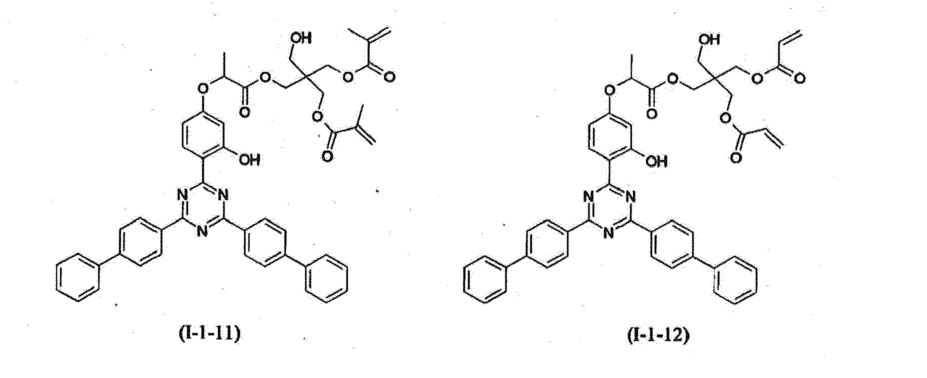

6. Composition de revêtement, **caractérisée en ce qu'**elle contient au moins un des composés selon au moins l'une quelconque des revendications 1 à 5 et au moins un solvant organique.

7. Composition de revêtement selon la revendication 6, **caractérisée en ce qu'**elle contient au moins un initiateur, de préférence au moins un photoinitiateur.

8. Composition de revêtement selon la revendication 6 ou 7, **caractérisée en ce qu'**elle contient au moins un stabilisateur du groupe des stabilisateurs HALS (Hindered Amine Light Stabiliser).

9. Composition de revêtement selon au moins l'une quelconque des revendications 6 à 8, **caractérisée en ce qu'**elle contient au moins un diluant réactif ou au moins un acrylate de polyester ou un acrylate d'uréthane aliphatique oligomère.

10. Composition de revêtement selon au moins l'une quelconque des revendications 6 à 9, **caractérisée en ce qu'**elle contient en outre un ou plusieurs additifs supplémentaires choisis dans le groupe contenant les stabilisateurs, les agents d'écoulement, les additifs de surface, les pigments, les colorants, les promoteurs d'adhésion, les absorbeurs IR et les absorbeurs UV différents des composés de formule générale (I).

11. Procédé de revêtement de substrats, **caractérisé en ce que**
A) une couche d'une composition de revêtement selon au moins l'une quelconque des revendications 6 à 10 est appliquée sur le substrat, puis
B) au moins une partie du solvant est éliminé de la couche obtenue selon l'étape A), puis
C) la couche obtenue selon l'étape B) est durcie.

12. Revêtement fabriqué à partir d'une composition de revêtement selon au moins l'une quelconque des revendications 6 à 10.

13. Article, **caractérisé en ce qu'**il contient au moins un substrat et au moins un revêtement fabriqué à partir d'au moins une composition de revêtement selon au moins l'une quelconque des revendications 6 à 10.

14. Article selon la revendication 13, **caractérisé en ce que** le substrat est un corps moulé, un extrudat ou un co-extrudat contenant un ou plusieurs plastiques thermoplastiques.

15. Feuille, plaque, plaque alvéolaire, vitre de phare, vitrage automobile ou vitrage architectural selon la revendication 13 ou 14.
